# EUROPEAN PATENT APPLICATION

(11) **EP 2 108 698 A1**
(43) Date of publication of application: **14.10.2009**
(21) Application number: 07851046.8
(22) Date of filing: 21.12.2007
(51) Int. Cl.: C12N 15/09, A61K 38/00, A61P 35/00, C07K 7/04

(54) **VASCULAR ENDOTHELIAL CELL-BINDING PEPTIDE**

(30) Priority: 09.01.2007 JP 2007001294; 07.09.2007 JP 2007232443
(71) Applicant: Toray Industries, Inc., Tokyo 103-8666 (JP)
(72) Inventor: KOYAMATSU, Yuichi, Kamakura-shi Kanagawa 248-8555 (JP); MICALLEF, Mark, Kamakura-shi Kanagawa 248-8555 (JP); IDA, Nobuo, Kamakura-shi Kanagawa 248-8555 (JP); KOIWA, Masakazu, Kamakura-shi Kanagawa 248-8555 (JP); KAKIZAWA, Yoshinori, Kamakura-shi Kanagawa 248-8555 (JP)
(74) Representative: Denison, Christopher Marcus
(86) International application number: PCT/JP2007/074640
(87) International publication number: WO 2008/084652

(57) **Abstract**

A peptide consisting of an amino acid sequence represented by any one of SEQ ID Nos. 1 to 76 of the present invention, a peptide consisting of an amino acid sequence in which one or several amino acids are substituted, deleted, inserted or added in these amino acid sequences, and having an ability to bind to, or to be taken into an activated vascular endothelial cell, or a peptide containing the above peptide as a partial sequence, and having an ability to bind to, or to be taken into an activated vascular endothelial cell is a novel peptide ligand specifically binding to a neovascular endothelial cell, and such a ligand can be effectively used for treating and diagnosing a disease accompanying vascularization such as a solid tumor.

## Description

### Technical Field

The present invention relates to a peptide having affinity for a vascular endothelial cell. More particularly, the present invention relates to a peptide having a specific binding property for an activated vascular endothelial cell.

### Background Art

A blood vessel has an important role in transporting various nutrients including oxygen, and cells to tissues of a whole body as is well known. Vascularization is a fundamental process for forming a new blood vessel, and this process is indispensable in many normal physiological phenomena such as ontogenesis, tissue growth, and wound healing. In addition, vascularization is also important in manifestation of some pathological conditions and, particularly, a role in proliferation and metastasis of a solid tumor is attracting attention. Besides a tumor, relationship of vascularization with various diseases such as arthritis, psoriasis, diabetic retinopathy, and age-related macular degeneration is known.

In a solid tumor, a phenomenon in which a cancer cell forms a new blood vessel in a tumor tissue for obtaining supply of oxygen, nutrient and the like necessary for proliferating the cancer cell itself is widely known, and it has been clarified that various vascularization promoting factors necessary for this vascularization are produced by a tumor cell. An attempt to suppress vascularization in a tumor tissue using a neutralizing antibody for a vascular endothelial growth factor (VEGF) which is one of factors of inducing such vascularization has been put into practice as an antibody drug and clinical use thereof has been initiated in recent years, and it is reported that the expected anti-tumor effect is exhibited (Non-patent Document 1).

As treatment of a tumor targeting a new blood vessel in a tumor tissue, in addition to the aforementioned method of inhibiting a vascular endothelial growth factor, a method of specifically delivering a drug to a neovascular endothelial cell, and causing suppression of a disorder or proliferation of this endothelial cell is considered. In addition, it can be expected to increase the concentration of a drug acting on a cancer cell to obtain the anti-cancer effect by increasing the concentration of an anti-cancer agent at the periphery of a neovasclular endothelial cell. In such an approach, it is an important point to obtain a ligand which specifically binds to a neovascular endothelial cell. As this neovascular endothelial cell-specific ligand, some peptides which bind to a VEGF receptor or av integrin, an increase in expression of which is reported in a neovascular endothelial cell, are reported (Non-patent Document 2), but little peptides have been confirmed to have sufficient efficacy in vivo, and the study is at an initial stage under the current circumstances. In a neovascular endothelial cell, in addition to the aforementioned molecules, it is thought that there are a plurality of unknown molecules whose expression is increased, and it is expected to obtain a ligand having higher efficacy by searching a ligand for such unknown molecules.
Non-patent Document 1: Herbert Hurvitz and 14 others, "Bevacizumab plus Irinotecan, Fluorouracil, and Leucovorin for Metastatic Colorectal Cancer", New Engl J Med, 2004, vol. 350, pp. 2335-2342
Non-patent Document 2: Arap Wadih and 2 others, "Cancer Treatment of Targeted Drug Delivery to Tumor Vasculature in a Mouse Model", Science, 1998, vol. 279, pp. 377-380

### Disclosure of the Invention

### Problems to be Solved by the Invention

An object of the present invention is to provide a novel peptide ligand which specifically binds to a neovascular endothelial cell.

### Means for Solving the Problems

In order to overcome the aforementioned problems, the present inventors studied screening of a peptide sequence which binds to an activated human vascular endothelial cell cultured by adding a culture supernatant of a tumor cell strain, that is, a model cell of a tumor neovascular endothelium, but does not bind to a human vascular endothelial cell cultured without adding a culture supernatant of a tumor cell strain, that is, a model cell of a normal vascular endothelium, from a random library, and obtained a peptide having an objective nature.

That is, the present invention has the following essential features.
(1) A peptide of any one of (A), (B) and (C):
   (A) a peptide consisting of an amino acid sequence represented by any one of SEQ ID Nos. 1 to 76;
   (B) a peptide having a sequence in which one or several amino acids are substituted, deleted, inserted or added in an amino acid sequence represented by any one of SEQ ID Nos. 1 to 76, and having an ability to bind to, or to be taken into an activated vascular endothelial cell; and
   (C) a peptide containing the peptide of (A) or (B) as a partial sequence, and having an ability to bind to, or to be taken into an activated vascular endothelial cell.
(2) A nucleic acid encoding the peptide as defined in (1).
(3) A peptide binding body wherein the peptide as defined in (1) is bound to a hydrophilic polymer.
(4) A pharmaceutical composition containing the peptide as defined in (1).
(5) A method of accumulating a drug in a new blood vessel, including using the peptide as defined in (1).

### Effects of the Invention

The peptide of the present invention can be expected to accumulate specifically in a new blood vessel present in a diseased tissue such as a solid tumor, and this nature can be employed in treatment or diagnosis of various diseases accompanied with vascularization.

### Brief Description of the Drawings

Fig. 1 is a drawing showing results of measurement of binding of each phage clone to an activated HUVEC and a non-activated HUVEC by an ELISA method.
Fig. 2 is a drawing substituting for a photograph showing results of observation of a clone 6 with a confocal fluorescent microscope.
Fig. 3 is a drawing substituting for a photograph of observation of uptake of a fluorescently labeled peptide-BSA conjugate into a HUVEC and a HeLa cell with a confocal laser microscope.
Fig. 4 is a drawing substituting for a photograph of observation of a tumor tissue section after immunostaining 24 hours after administration of a fluorescently labeled multiarm PEG-peptide conjugate to a mouse transplanted cancer cells, with a fluorescent microscope.
Fig. 5 is a drawing showing results of quantitation of an amount of intracellular uptake of doxorubicin, upon reaction of a peptide-modified liposome encapsulating doxorubicin on a HUVEC.

### Best Mode for Carrying Out the Invention

The peptide of the present invention is a peptide consisting of an amino acid sequence shown in SEQ ID Nos. 1 to 76, or a peptide having an amino acid sequence in which one or several amino acids are substituted, deleted, inserted or added in an amino acid sequence represented by any one of SEQ ID Nos. 1 to 76, and having an ability to bind to, or to be taken into an activated vascular endothelial cell.

Herein, a substituted, inserted or added amino acid may be a molecule having a carboxy group and an amino group regardless of the presence or absence in the natural world, and may be an amino acid which has been subjected to post-translation modification usually seen in a living body, such as hydroxylation, phosphorylation, or glycosylation. Preferably, the amino acid has an amino acid sequence consisting of a natural amino acid and an optical isomer thereof which are usually present in a mammal cell, and examples thereof include amino acid sequences consisting of arginine (Arg), lysine (Lys), aspartic acid (Asp), asparagine (Asn), glutamic acid (Glu), glutamine (Gln), histidine (His), proline (Pro), tyrosine (Tyr), tryptophan (Trp), serine (Ser), threonine (Thr), glycine (Gly), alanine (Ala), methionine (Met), cysteine (Cys), phenylalanine (Phe), leucine (Leu), valine (Val), and isoleucine (Ile).

The number of amino acids to be substituted, deleted, inserted or added is preferably 1 to 7, further preferably 1 to 5, and further preferably 1 to 3. In a sequence mutated by substitution, deletion or insertion, a continuous arrangement of preferably 4 or more amino acids, more preferably 6 or more amino acids of the original amino acid sequence represented by any one of SEQ ID Nos. 1 to 76 is conserved.

In addition, the present invention includes a peptide containing, as a partial amino acid sequence, the aforementioned peptide, that is, a peptide consisting of an amino acid sequence shown in SEQ ID Nos. 1 to 76, or a peptide having an amino acid sequence in which one or several amino acids are substituted, deleted, inserted or added in an amino acid sequence represented by any one of SEQ ID Nos. 1 to 76, and having an ability to bind to, or to be taken into an activated vascular endothelial cell, and having an ability to bind to, or to be taken into an activated vascular endothelial cell.

The peptide referred to in the present invention broadly means a substance in which two or more amino acids are linked with an amide bond, and includes a substance usually called a protein, such as a substance in which a few tens to a few hundreds or a few thousands of amino acids are linked.

The vascular endothelial cell referred to in the present invention is a cell constituting an inner surface of a blood vessel, and it is preferable to use a human endothelial cell or other mammal endothelial cells. The vascular endothelial cell includes, without limitation, a human umbilical vein endothelial cell (HUVEC), a human dermal microvascular endothelial cell and a human lung microvascular endothelial cell (HMVEC).

The activated vascular endothelial cell referred to in the present invention refers to an endothelial cell cultured in the presence of various factors which are known to promote vascularization, for example, a vascular endothelial growth factor (VEGF), a fibroblast growth factor (FGF), a platelet-derived growth factor (PDGF), an angiopoietin and the like, or a mixture of these factors. Alternatively, the activated vascular endothelial cell refers to an endothelial cell cultured in the presence of an arbitrary factor produced by a tumor cell. An endothelial cell cultured in the presence of a factor produced by a tumor cell, for example, an endothelial cell cultured by adding a culture supernatant of a tumor cell to a medium can be expected to highly mimic a nature of an endothelial cell constituting a new blood vessel induced in a solid tumor tissue, and is particularly preferably used. As a tumor cell used herein, various internal organ tumor cells derived from a human or other mammals can be widely used. Examples thereof include HuH-7 cells derived from liver cancer, MCF-7 cells derived from breast cancer, OVCAR-3 cells derived from ovary cancer, KSIMM cells derived from Kaposi's sarcoma, LOVO cells derived from colon cancer, MKN-45 cells derived from stomach cancer, DU145 cells derived from prostate cancer, A549 cells derived from lung cancer, U-87MG cells derived from brain tumor, SK-MEL-5 cells derived from skin cancer, T24 cells derived from bladder cancer, PANC-1 cells derived from pancreas cancer, and GRC-1 cells derived from kidney cancer.

The time for culturing an endothelial cell in the presence of these vascularization promoting factors or factors produced by a tumor cell is not particularly limited, but as conditions under which a cell undergoes a sufficient action and is not excessively proliferated, 2 hours or longer and 72 hours or shorter is preferable, and 8 hours or longer and 48 hours or shorter is further preferable.

As a method of culturing in the presence of an arbitrary factor produced by a tumor cell, there are a method of culturing a tumor cell alone, recovering the culture supernatant, and adding the supernatant to a medium of an endothelial cell, and a method using a filter through which a cell does not permeate but various proteins and low-molecular substances can permeate, and placing a vascular endothelial cell into one and a tumor cell into another, followed by culturing. In order to obtain an activated endothelial cell having a constant nature at good reproductivity, preferable is a method of culturing a tumor cell alone, recovering the culture supernatant, and adding the supernatant to a medium of an endothelial cell.

The peptide having an ability to bind to a vascular endothelial cell referred to in the present invention means a peptide which can stay on a surface of the vascular endothelial cell by chemical or physical interaction with a molecule (cell surface molecule) present on a surface of the cell, and also referred to as a vascular endothelial cell binding peptide. The cell surface molecule referred to herein includes, without limitation, lipids, proteins, and polysaccharides. In addition, the interaction may be a single cell surface molecule or a plurality of cell surface molecules.

The peptide having an ability to be taken into a vascular endothelial cell referred to in the present invention means a peptide having a nature that, after the peptide is contacted with the cell, it can pass through a cell membrane and enter the interior of the cell. The passing means includes, without limitation, endocytosis. In the invention, a peptide having an ability to be taken into a vascular endothelial cell is also included in a vascular endothelial cell binding peptide.

The ability to bind to, or the ability to be taken into a vascular endothelial cell, of the peptide of the present invention can be assessed, for example, using a confocal laser microscope observation, flow cytometry, or a fluorescent intensity meter. For example, one example of an assessing method using a confocal laser microscope will be described without limitation. First, a 96-well microplate (glass bottom plate, Nunc No. 164588) is treated with 100 µl of a 1% acidic collagen solution for 10 minutes in advance, washed with 120 µl of PBS once, seeded with 100 µl of 1 × 10⁵/ml HUVEC cells, and cultured under the condition of 37°C and 5% CO₂ for 2 days. Upon use, cells are washed with 150 µl of a medium (EBM-2 medium containing a 2% fetal bovine serum (containing a growth factor, Cambrex Bio Science)) once, 50 µl of the same medium, and 50 µl of a solution obtained by diluting a fluorescently labeled peptide or a fluorescently labeled peptide-polymer conjugate with a medium to the peptide concentration of 20 µM, and culturing is performed under the condition of 37°C and 5% CO₂ for 8 hours (the peptide concentration is 10 µM at reaction with cells). Cells after the reaction are observed using a confocal laser microscope (Olympus FV1000), and whether fluorescence derived from a peptide is present on a surface of a cell or the interior of a cell or not is investigated, thereby, the ability to bind to, or to be taken into a vascular endothelial cell can be assessed.

The peptide of the present invention can be produced by a general chemical synthesis method. The production method includes peptide synthesis methods according to a usual liquid phase method and a solid phase method. Such a peptide synthesis method includes a stepwise elongation method of sequentially binding each amino acid one by one to extend a chain based on amino acid sequence information, and a fragment condensation method of synthesizing a fragment consisting of a few amino acids in advance and then, coupling-reacting each fragment. Synthesis of the peptide of the present invention may be carried out according to any of these methods.

The condensation method adopted in the peptide synthesis may be according to the known various methods. Examples thereof include an azido method, a mixed acid anhydride method, a DCC method, an active ester method, an oxidation reduction method, a DPPA (diphenylphosphorylazide) method, a method of adding 1-hydroxybenzotriazole, N-hydroxysuccinimide, N-hydroxy-5-norbornene-2,3-dicarboxyimide or the like to DCC, and a Woodward method. The solvent which can be utilized in these methods can be arbitrarily selected from general solvents which are well-known to be used in this kind of peptide condensation method. Examples thereof include solvents such as dimethylformamide (DMF), dimethyl sulfoxide (DMSO), hexamethylphosphoroamide, dioxane, tetrahydrofuran (THF) and ethyl acetate, and mixed solvents of these solvents.

Upon the peptide synthesis reaction, a carboxyl group in an amino acid or a peptide not involved in a reaction can be protected as a lower alkyl ester such as a methyl ester, an ethyl ester or a tertiary butyl ester, or an aralkyl ester such as a benzyl ester, a p-methoxybenzyl ester or a p-nitrobenzyl ester. In addition, an amino acid having a functional group on a side chain, for example, a hydroxyl group of Tyr may be further protected with an acetyl group, a benzyl group, a benzyloxycarbonyl group, a tertiary butyl group or the like, if necessary. Further, for example, a guanidino group of Arg can be protected with a suitable protective group such as a nitro group, a tosyl group, a 2-methoxybenzenesulfonyl group, a methylene-2-sulfonyl group, a benzyloxycarbonyl group, an isobornyloxycarbonyl group or an adamantyloxycarbonyl group. A reaction of deprotecting these protective groups in an amino acid, a peptide and a finally obtained peptide of the present invention having the protective group can be performed by the conventional method, for example, a catalytic reduction method, or a method using liquid ammonia/sodium, hydrogen fluoride, hydrogen bromide, hydrogen chloride, trifluoroacetic acid, acetic acid, formic acid, methanesulfonic acid or the like.

Alternatively, the peptide of the present invention can be also prepared by the conventional method using a genetic engineering procedure. The thus obtained peptide of the present invention can be appropriately purified by the conventional method, for example, the method which is generally used in the field of peptide chemistry, such as an ion exchange resin, distribution chromatography, gel chromatography, affinity chromatography, high performance liquid chromatography (HPLC), or a countercurrent method.

Alternatively, the peptide of the present invention can be also used in the state of being bound to other substances. Binding may be chemical or physical, or a linker may be held between the present peptide and other substances. Specific examples of other substances to be bound include polymers, lipids, saccharides, and low-molecular compounds and, among them, from the viewpoint that blood retention is improved, and that the cluster effect due to high density modification can be expected, a hydrophilic polymer is preferable. Examples of the hydrophilic polymer referred to herein include chemically synthesized or synthetic polymers, as well as bio- (natural) polymers such as polysaccharides, nucleic acids and proteins and, among them, a synthetic polymer, particularly polyethylene glycol (PEG) and polyvinyl alcohol (PVA) are more preferably used and, more particularly, PEG is suitably used from the viewpoint of good blood retention.

Examples of a method of binding the peptide and the hydrophilic polymer include a method of performing binding utilizing a reactive group introduced into the hydrophilic polymer. As the reactive group to be introduced, groups which react with any of a SH group, an OH group, a COOH group, and a NH₂ group present in the peptide are preferable, and examples thereof include, without limitation, a maleimide group, an N-hydroxysuccinimide group, a dithiopyridine group, an NH₂ group, a COOH group, an OH group, and a SH group. It is known that PEG can be functionalized so that it contains a reactive group suitable for reacting with a functional group such as a -NH₂ group, a -COOH group, an -OH group, or a -SH group present in the peptide and, for example, when PEG in which a maleimide group is introduced into a terminus (e.g. Multiarm-PEG, SUNBRIGHT PTE-200 MA manufactured by NOF CORPORATION) is used, a peptide-PEG conjugate can be obtained by reacting the PEG with an -SH group present in the peptide.

In addition, the peptide of the present invention can be used as a pharmaceutical composition containing a biologically active drug and the peptide of the present invention. It is preferable that the peptide of the present invention and a drug in a pharmaceutical composition form a conjugate. As used herein, the conjugate indicates the state where two or more substances can be moved simultaneously, and examples thereof include a conjugate in which substances are bound with a covalent bond, a conjugate in which substances are bound electrostatically with an ion bond, and even in the case of the absence of a bond, a conjugate in which the other suppresses movement of the other due to a steric structure, and both can be moved. For example, formation of the conjugate in which a biologically active drug is encapsulated in a fine particle of a micelle, a liposome or a polymer obtained by modifying a surface of the peptide of the present invention is included.

As the kind of the drug, use of various drugs having the activity of suppressing or promoting proliferation of a cell is considered. In treatment targeting a tumor angiogenesis, various drugs such as doxorubicin, paclitaxel, cisplatin, oxaliplatin, 5FU, CPT-11, and mitomycin C, which are used as an anti-cancer drug can be used.

In addition, a so-called biopharmaceuticals such as a protein drug such as an antibody, or a nucleic acid drug such as a siRNA or an aptamer is also preferably used. When a protein is used as a drug, a fused protein can be also made and used as a conjugate of the peptide of the present invention, and a protein. In this case, the position to which the peptide of the present invention is bound is not particularly limited, and it is preferable that a peptide is presented on an outer side of a protein, and influence of the fused protein on the activity and the function is low, and the position is desirably an amino terminus or a carboxyl terminus.

When a fused protein with the peptide of the present invention is produced, it can be produced by a general chemical synthesis method. To show an example, there are a method of mixing the peptide of the present invention and a protein drug, and adding a condensing agent to bind them, and a method using a peptide synthesizer (e.g. Applied Biosystems Model 433). Alternatively, the fused protein can be also produced by the conventional method using a genetic engineering procedure based on nucleotide sequence information. Examples thereof include a method of producing the fused protein by incorporating nucleotide sequences encoding the peptide of the present invention and a protein to be fused into a gene expression vector having a protein expressing promoter.

Examples of the pharmaceutical composition of the present invention include a powder form consisting of a pharmaceutically acceptable additive in addition to the peptide of the present invention and a drug, a liquid form consisting of a mixture of a medium such as water and a pharmaceutically acceptable base in addition to the medium, and a form solidified or semi-solidified with a combination with a pharmaceutically acceptable base. Examples of the base include conventional various organic or inorganic substances as a preparation material, and examples thereof include excipients, lubricants, binders, disintegrating agents, solvents, solubilizers, suspending agents, isotonic agents, buffers, soothing agents, and absorption promoters.

The peptide obtained in the present invention can be expected to specifically accumulate in a new blood vessel present in a diseased tissue such as a solid tumor and, using this nature, the peptide can be used in treatment and diagnosis of various diseases. Examples of a cancer kind in which vascularization should be inhibited include breast cancer, skin cancer, colorectum cancer, pancreas cancer, prostate cancer, lung cancer, non-small cell lung cancer, ovary cancer, liver cancer, brain tumor, esophagus cancer, bladder cancer, uterocervical cancer, fatty sarcoma, epithelium cancer, kidney cell cancer, gallbladder adenocarcinoma, parotid cancer, melanoma, lymphoma, glioma, endometrioma, and multi-drug resistant cancer. In addition, examples of a disease other than a cancer are not limited to, but include diseases such as chronic inflammatory such as rheumatoid arthritis, psoriasis, or osteoarthritis, and retinopathy such as age-related macular degeneration, diabetic retinopathy, or neovascular glaucoma.

By administering a pharmaceutical composition containing both of a biologically active drug and the peptide of the present invention to a body, it becomes possible to accumulate the drug in a new blood vessel of a diseased tissue. The method of administering the pharmaceutical composition is not particularly limited, and examples thereof include a method by injection, oral administration, pulmonary administration, intranasal administration, and ocular instillation administration. Administration by injection is preferable and, particularly, administration into a vein or artery is a preferable method since a drug can efficiently reach a new blood vessel diseased site.

The dose and number of times of administration upon administration of the pharmaceutical composition of the present invention to a body can be appropriately selected depending on a drug to be combined with the peptide, an administration form, an age and a weight of a patient, and severity of a symptom, and the composition is administered in the range of usually 0. 1 µg to 10 g, preferably 1 µg to 1000 mg per adult male a day.

### Examples

The following examples illustrate the present invention in detail, but the present invention is not limited to these examples.

### Example 1 Screening of activated vascular endothelial cell-binding phage

### <Method>

### 1. Preparation of tumor cell culture supernatant

A human liver cancer cell strain HuH-7 was diluted with 7 ml of a DMEM medium containing a 5% fetal bovine serum, placed into a 25 cm² culturing flask, and cultured at 37°C under the presence of 5% CO₂ for 24 hours. Culturing was performed in the state where the cell density was about 70% confluent. The culture solution was recovered, and centrifuged at 1000 rpm for 5 minutes to recover the supernatant. The recovered culture supernatant was stored at -30°C until use.

### 2. Preparation of activated normal human umbilical vein endothelial cell (HUVEC)

Normal human umbilical vein endothelial cells (HUVEC) (Cambrex Bio Science) used in phage library screening were seeded on a 12-well plate in an EBM-2 medium (Cambrex Bio Science, containing a growth factor) containing a 2% fetal bovine serum in advance at the density of 1 × 10⁴/ml, 1 ml/well, and were cultured for 48 hours. The culture was washed with PBS three times, 0.5 ml of the HuH-7 liver cancer cell culture supernatant prepared as described above, and 0.5 ml of an EBM-2 medium (not containing a growth factor) were added to one well, and 0.5 ml of a DMEM medium containing 5% FCS and 0.5 ml of an EBM-2 medium (not containing a growth factor) were added to the other well, followed by culturing for 24 hours. Cells obtained by culturing by adding the HuH-7 liver cancer cell culture supernatant were used as a "HuH-7 supernatant-cultured HUVEC", and cells obtained by culturing by adding a DMEM medium were used as a "DMEM-cultured HUVEC", in the following screening.

### 3. Screening from phage library

As the phage peptide library, the M13 line phage library exhibiting a 15-mer or 12-mer random amino acid sequence as a fused protein of a phage pIII protein was used. The DMEM-cultured HUVEC prepared in the above item 2. was washed with PBS three times, and 1 ml of a 0.1% bovine serum albumin (BSA)/phosphate buffered saline (PBS) containing 1.2 × 10¹⁰ TU (transforming unit) phages was added, followed by a reaction at room temperature for 30 minutes. The reaction solution (unbound phages) was recovered and, now, added to the HuH-7 supernatant-cultured HUVEC (pre-washed with PBS three times) prepared in the above item 2., followed by a reaction at room temperature for 30 minutes. Cells after the reaction were washed with PBS three times, and 0.5 ml of an acidic eluant (0.1 M glycine hydrochloride (pH 2.4) containing 0.1% BSA) was added to perform a reaction for 1 minute, to dissociate bound phages. The eluant was recovered, and centrifuged at 1200 rpm for 5 minutes, and 125 µl of 1 M Tris hydrochloride (pH 8.0) was added to perform neutralization. This solution was added to the DMEM-cultured HUVEC (pre-washed with PBS three times); a reaction was performed at room temperature for 30 minutes, and the supernatant was recovered, thereby, phages selectively binding to the HuH-7 supernatant-cultured HUVEC were obtained.

The resulting phage solution was infected on Escherichia coli in a logarithmic proliferation phase, and this was cultured, thereby, phages were amplified. Phages produced in the culture solution were centrifugation-recovered by adding a PEG/NaCl solution (20% PEG8000 containing 2.5 M sodium chloride) for precipitation, and thereby phages were purified.

Letting isolation of HuH-7 supernatant-cultured HUVEC-binding phages by the aforementioned series of procedures, and amplification with Escherichia coli infection to be one cycle, a total of 3 cycles of screening was performed. A phage recovery solution after 3 cycles was seeded on an LB-agar plate, and cultured at 37°C overnight, a few tens of colonies were randomly selected from the resulting colonies (single clone) and individually cultured, and the supernatant phages were purified by the aforementioned PEG/NaCl precipitation method, thereby, a purified phage clone was obtained.

### 4. Analysis of cell binding property of phage clone by Cell ELISA method

A HUVEC (0.1 ml) was seeded on a 96-well plastic plate in an EBM-2 medium containing a 2% fetal bovine serum at the density of 3 × 10⁴/ml, cultured at 37°C for 24 hours, washed with 150 µl of PBS, 0.05 ml of the HuH-7 liver cancer cell culture supernatant and 0.05 ml of an EBM-2 medium (not containing a growth factor) were added to an activated HUVEC-prepared well, and 0.05 ml of a DMEM medium containing 5% FCS and 0.05 ml of an EBM-2 medium (not containing a growth factor) were added to a non-activated HUVEC-prepared well, followed by culturing at 37°C for 24 hours. After cells were washed with PBS, phage clones diluted 50-fold with PBS containing 0.1% BSA were added, followed by a reaction at room temperature for 30 minutes. Cells after the reaction were washed with 150 µl of PBS three times, 50 µl of PBS containing 0.5% paraformaldehyde was added, followed by a fixation reaction at room temperature for 30 minutes. After the cells were washed with PBS containing 0.05% Tween 20 three times, 50 µl of an HRP-labeled anti-M13 phage antibody (Amersham) diluted 5000-fold with PBS containing 0.1% BSA was added, followed by a reaction at room temperature for 60 minutes. After the product was washed with PSB containing 0.05% Tween 20 three times, 100 µl of an HRP enzyme substrate (acetate citrate buffer (pH 4.5) containing 0.2 µl/ml of aqueous hydrogen peroxide and 0.08 mg/ml of tetramethylbendizine) was added, followed by a reaction at room temperature for 3 to 5 minutes. Thereafter, 50 µl of 1 N sulfuric acid was added to stop the reaction. Using a microplate reader, an absorbance at 450 nm (reference wavelength 595 nm) was measured.

### <Results>

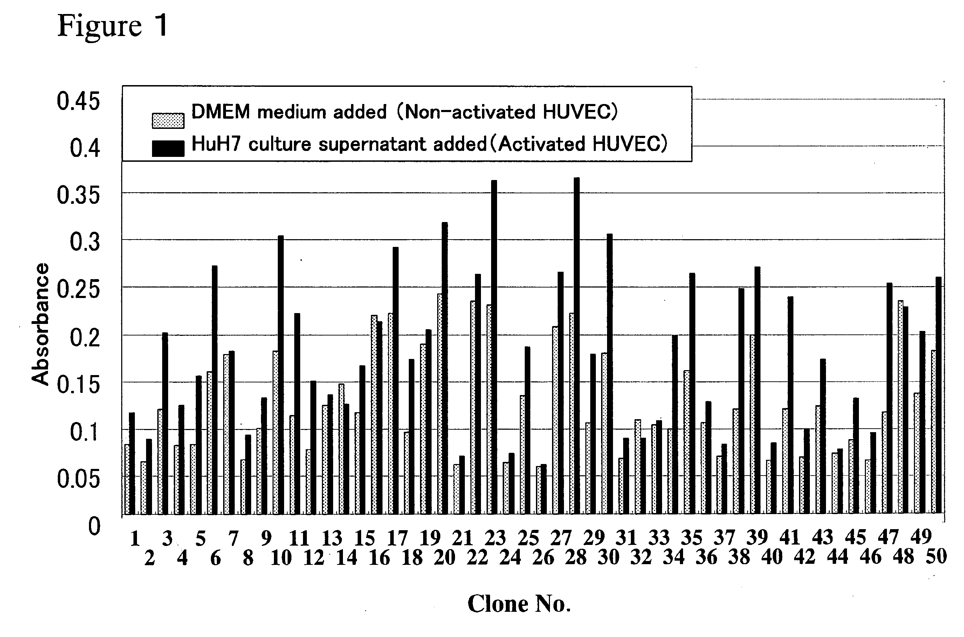

Results of measurement of an absorbance of each phage clone are shown in Fig. 1. A greater absorbance indicates that the property of binding to a HUVEC is better. It was recognized that, of 50 clones, a half or more of clones have a better property of binding to an activated HUVEC (in the drawing, added with HuH-7-culture supernatant) as compared with binding to a non-activated HUVEC (in the drawing, added with DMEM medium).

### 5. Analysis of sequence of phage clone

Screening of Examples 1-1 to 4 was repeatedly performed a few times, a total of 68 kinds of clones whose property of binding to an activated HUVEC was recognized as good were selected, infected with Escherichia coli, and amplified, a nucleotide sequence of a region containing a random peptide sequence of a phage pIII protein was analyzed, thereby, a presented peptide was sequenced.

### <Results>

A total 68 kinds of peptide sequences represented by SEQ ID Nos. 1 to 14 and 22 to 75 were obtained (Table 1).

**[Table 1-1]**

| Peptide | | Sequence | Clone ID |
|---|---|---|---|
| Sequence-1 | Vascular endothelial cell-binding Peptide | Asp Arg Arg Val Ser Leu Arg Ile Pro Phe Ser Ile Leu His Arg | clone6 |
| Sequence-2 | Vascular endothelial cell-binding Peptide | Ser Ser Tyr Lys Trip Leu Leu Ala Asp Tyr Pro Gln Arg Leu Leu | clone23 |
| Sequence-3 | Vascular endothelial cell-binding Peptide | Ser His Val Ala Ser Leu Leu Pro Ala Leu Ala Lys Gly Gly Arg | clone11 |
| Sequence-4 | Vascular endothelial cell-binding Peptide | Asn Arg Gly Tyr Ser Ser Phe Gln Thr Phe Gly His Leu Leu Leu | clone12 |
| Sequence-5 | Vascular endothelial cell-binding Peptide | Arg Trp Leu Pro Val Leu Ser Leu Lys Tyr Val Lys Trp Glu His | clone20 |
| Sequence-6 | Vascular endothelial cell-binding Peptide | Arg Leu Trp Met Leu Arg Ala Asp Phe Val Ser Ser Arg Thr Asp | clone25 |
| Sequence-7 | Vascular endothelial cell-binding Peptide | Leu Gln Phe Pro Leu Val Ile Pro Ile Leu Ser Ile Tyr Thr Ala | clone28 |
| Sequence-8 | Vascular endothelial cell-binding Peptide | Lys Lys Asp Leu Ile Asn Leu Ala Val Gly Phe Ile Asp Ser Phe | clone29 |
| Sequence-9 | Vascular endothelial cell-binding Peptide | Ile Thr Leu Val His Pro Gly Ala Tyr Phe His Phe Leu Leu Asp | clone3 |
| Sequence-10 | Vascular endothelial cell-binding Peptide | Tyr Trp Phe Ser Ile Leu Gly Asp Leu Leu Pro Glu Val Ala Asn | clone5 |
| Sequence-11 | Vascular endothelial cell-binding Peptide | Gly Met Arg Pro Arg Leu Ala Ser Asn Ser Gly Met Ser Asn Leu | clone34 |
| Sequence-12 | Vascular endothelial cell-binding Peptide | Thr Leu Pro Ser Pro Leu Ala Leu Leu Thr Val His | PhD clone1-14 |
| Sequence-13 | Vascular endothelial cell-binding Peptide | Ser Leu Thr Val Pro Phe Leu Pro Leu Tyr Val Pro | PhD clone3-30 |
| Sequence-14 | Vascular endothelial cell-binding Peptide | Ala Lys Val Gly Thr Gln Leu Phe Leu Ile His Ala Ser Ile Phe | clone38 |
| Sequence-22 | Vascular endothelial cell-binding Peptide | Ser Tyr Leu Asn Ser Lys Leu Leu Pro Pro Ser Ala Leu Thr Gly | ST-1 |
| Sequence-23 | Vascular endothelial cell-binding Peptide | Ser Gly Ile Ser Trp Pro Leu Glu Ala Leu Ala Leu Trp Leu Leu | ST-2 |
| Sequence-24 | Vascular endothelial cell-binding Peptide | Gly Ile Ala Trp Thr Asn Arg Ile Val Ser Asp Ala Val Glu Pro | ST-3 |
| Sequence-25 | Vascular endothelial cell-binding Peptide | Ala Asn Asn Pro Trp Gln Glu Met Ile Ser Tyr Glu Lys Ile His | ST-4 |
| Sequence-26 | Vascular endothelial cell-binding Peptide | Ser Arg Val Pro Gly Met Tyr Asn Asp Gln Arg Ala Thr Phe Phe | ST-5 |
| Sequence-27 | Vascular endothelial cell-binding Peptide | Cys Tyr Leu Thr Met Thr Ala Val Ser Arg Ser Gln Tyr Ser Leu | ST-6 |
| Sequence-28 | Vascular endothelial cell-binding Peptide | Thr Thr Arg Val Trp Leu Asp Gln Met Val Glu Pro Gly Asn Glu | ST-7 |
| Sequence-29 | Vascular endothelial cell-binding Peptide | Asn Leu His Trp Thr Leu Thr Phe Ser Lys Val Pro Thr Gly Glu | ST-8 |
| Sequence-30 | Vascular endothelial cell-binding Peptide | Gly Val Gly Gln Leu Ala Met Thr Leu Ser Gly Arg Gly Ser His | ST-9 |

**[Table 1-2]**

| | | | |
|---|---|---|---|
| Sequence-31 | Vascular endothelial cell-binding Peptide | Ser Arg Thr Pro Asn Asn Ile Met Ala Val Asn Ser His Ser Arg | ST-10 |
| Sequence-32 | Vascular endothelial cell-binding Peptide | Ser Met Val Met Asp Leu Arg Gly Lys Phe Pro Ser Val Arg Val | ST-11 |
| Sequence-33 | Vascular endothelial cell-binding Peptide | Ser Lys Ala Pro Ser Ttp Asp Leu Pro Lys Thr Gly Gly Glu Ile | ST-12 |
| Sequence-34 | Vascular endothelial cell-binding Peptide | Ala Arg Arg Pro Ala Val Lys Ala Val Thr Thr Asp Ser Tyr Gly | ST-13 |
| Sequence-35 | Vascular endothelial cell-binding Peptide | Ser Arg Met Pro Met Gly Val His Asp Thr Thr Ala Leu Lys Trp | ST-14 |
| Sequence-36 | Vascular endothelial cell-binding Peptide | Gln Ser Leu Ile Tyr Ser Ser Leu Phe Pro Arg Arg Ser Trp Phe | ST-15 |
| Sequence-37 | Vascular endothelial cell-binding Peptide | Met Thr Leu Ser Met Ala Arg Thr Ala Arg Asp Ile Gly Thr Gly | ST-16 |
| Sequence-38 | Vascular endothelial cell-binding Peptide | Ser Pro Arg Pro Leu Pro Ile Ile Thr Pro Phe Pro | PhD clone1-4 |
| Sequence-39 | Vascular endothelial cell-binding Peptide | His Val Thr Ser Tyr Asp Ala Trp Ala Pro Asp Pro | PhD clone1-5 |
| Sequence-40 | Vascular endothelial cell-binding Peptide | Asp Tyr Pro Lys Ala Pro Gly Ser His Pro Arg Thr | PhD clone1-21 |
| Sequence-41 | Vascular endothelial cell-binding Peptide | Thr Ser Lys Phe Pro Ser Thr Asp Leu Ala Arg Leu | PhD clone1-29 |
| Sequence-42 | Vascular endothelial cell-binding Peptide | Val Gly Asn Ser Asp Thr Thr Gly Thr Ser Arg Val | PhD clone1-30 |
| Sequence-43 | Vascular endothelial cell-binding Peptide | Ala Thr Trp Ser His His Leu Ser Ser Ala Gly Leu | PhD clonel-36 |
| Sequence-44 | Vascular endothelial cell-binding Peptide | Met Gly Tyr Asp Phe Thr Arg Ser Pro Leu Thr Trp | PhD clone2-1 |
| Sequence-45 | Vascular endothelial cell-binding Peptide | Met Ala Asn Leu Thr Gly Ser Gly Thr His Asn Leu | PhD clone2-6 |
| Sequence-46 | Vascular endothelial cell-binding Peptide | His Leu Ser Ser Arg Asp Leu Ser Leu Thr Ser Leu | PhD clone2-8 |
| Sequence-47 | Vascular endothelial cell-binding Peptide | Trp Gly Ile Ser Ile Thr Asn Pro Ala Ala Leu Ser | PhD clone2-9 |
| Sequence-48 | Vascular endothelial cell-binding Peptide | Asp Val Asn Lys Leu Leu Leu Arg Leu Pro Val Thr | PhD clone2-12 |
| Sequence-49 | Vascular endothelial cell-binding Peptide | Ala Ile Pro Ile Gly Thr Leu Pro Lys Ile His Leu | PhD clone2-22 |
| Sequence-50 | Vascular endothelial cell-binding Peptide | Tyr Pro Gln Ala Ser Leu Ser Thr Phe Lys Pro Leu | PhD clone2-23 |
| Sequence-51 | Vascular endothelial cell-binding Peptide | Asn Val Phe Arg His Ala Ile Glu Gln Arg Thr Pro | PhD clone2-28 |
| Sequence-52 | Vascular endothelial cell-binding Peptide | Ser Phe Tyr Asn Ala Asp Ser Ser Val Ser Arg Leu | PhD clone2-30 |
| Sequence-53 | Vascular endothelial cell-binding Peptide | Trp Ser Pro Ser Gln Phe Lys Leu Asp Met Pro His | PhD clone3-2 |
| Sequence-54 | Vascular endothelial cell-binding Peptide | Asn Ile Ala Lys Thr Ser Asn Ser Ser His Leu Pro | PhD clone3-7 |
| Sequence-55 | Vascular endothelial cell-binding Peptide | Gly Pro Met Leu Asp Met Ala Leu Gly Pro Ala Pro | PhD clone3-14 |

**[Table 1-3]**

| | | | |
|---|---|---|---|
| Sequence-56 | Vascular endothelial cell-binding Peptide | Lys Ser Phe Leu Tyr Thr Pro Ala Thr Val Thr His | PhD clone3-16 |
| Sequence-57 | Vascular endothelial cell-binding Peptide | Tyr Ala Thr Val His Thr Pro Leu Ala Trp Leu Pro | PhD clone3-18 |
| Sequence-58 | Vascular endothelial cell-binding Peptide | Trp Met Pro Thr Lys Pro His Leu His Asn Leu Gly | PhD clone3-24 |
| Sequence-59 | Vascular endothelial cell-binding Peptide | Glu Asn Pro Ser Pro Tyr Ile Pro Ile Pro Leu Thr | PhD clone3-25 |
| Sequence-60 | Vascular endothelial cell-binding Peptide | Glu Val Pro Leu Thr Gln Phe Leu Trp His Gln Leu | PhD clone3-35 |
| Sequence-61 | Vascular endothelial cell-binding Peptide | Met Ser Lys Asp Gln Pro Ser Phe Phe Arg Thr Ser | PhD clone3-39 |
| Sequence-62 | Vascular endothelial cell-binding Peptide | His Ala Ala Tyr Leu Val Glu Trp Pro Gly Ala Gly | PhD clone3-42 |
| Sequence-63 | Vascular endothelial cell-binding Peptide | Leu Pro Val Gln Leu Pro Val Asn Ile Ser Pro Arg | PhD clone3-44 |
| Sequence-64 | Vascular endothelial cell-binding Peptide | Gln Leu Leu Asn Ala Leu Pro Phe Arg Leu Ala Tyr | PhD clone3-45 |
| Sequence-65 | Vascular endothelial cell-binding Peptide | Ala Met Gly His Gly Leu Ala Lys Val Thr Thr Arg | PhD clone3-47 |
| Sequence-66 | Vascular endothelial cell-binding Peptide | Thr Asn Ser His Ala Val Pro Leu Leu Pro Leu Leu | PhD clone3-49 |
| Sequence-67 | Vascular endothelial cell-binding Peptide | Asp Ser Thr Met Met Pro Thr Val Leu Ser Thr Leu | PhD clone3-50 |
| Sequence-68 | Vascular endothelial cell-binding Peptide | Val Ser Gly Ser Glu Thr His Thr Met Pro Leu Ala | PhD clone3-51 |
| Sequence-69 | Vascular endothelial cell-binding Peptide | His Lys Leu Ala Thr Ser Pro Trp Trp Pro Pro Ile | PhD clone3-53 |
| Sequence-70 | Vascular endothelial cell-binding Peptide | Ser Thr His His Arg His Tyr His Asp Thr Leu Ala | PhD clone3-54 |
| Sequence-71 | Vascular endothelial cell-binding Peptide | Phe Ala Ser Ser His Lys Gln Ile Pro Leu Gln Leu | PhD clone3-67 |
| Sequence-72 | Vascular endothelial cell-binding Peptide | Asp Ser Leu Tyr Thr Leu Phe Pro Arg Ser Ala Phe | PhD clone3-71 |
| Sequence-73 | Vascular endothelial cell-binding Peptide | His Asn Pro Ile Pro Tyr Thr Ala Pro Leu Leu Phe | PhD clone3-72 |
| Sequence-74 | Vascular endothelial cell-binding Peptide | Met Ile Asn Thr Lys Ile Pro Pro Phe Thr Arg Trp | PhD clone3-73 |
| Sequence-75 | Vascular endothelial cell-binding Peptide | His Ala Lys Ser Gln Pro Ile Asn Ser Phe Ser Met | PhD clone3-74 |

### Example 2 Confirmation of binding of phage to HUVEC with confocal fluorescent microscope

### <Method>

A HUVEC (0.1 ml) was seeded on a 96-well glass bottom plate (a plate obtained by adding a collagen solution in advance, washing and coating) in an EBM-2 medium containing a 2% fetal bovine serum at the density of 3 × 10⁴/ml, cultured at 37°C for 24 hours, and washed with 150 µl of PBS, 0.05 ml of the HuH-7 liver cancer cell culture supernatant and 0.05 ml of an EBM-2 medium (not containing a growth factor) were added to an activated HUVEC-prepared well, and 0.05 ml of a DMEM medium containing 5% FCS and 0.05 ml of an EBM-2 medium (not containing a growth factor) were added to a non-activated HUVEC-prepared well, followed by culturing at 37°C for 24 hours. Cells used were washed with PBS, phage clones (phage number: about 2 × 10⁹ TU/ml) diluted with PBS containing 0.1% BSA were added, followed by a reaction at room temperature for 30 minutes. Cells after the reaction were washed with 150 µl of PBS three times, 100 µl of PBS containing 4% paraformaldehyde was added, followed by a fixation reaction at 37°C for 10 minutes. After the cells were washed with PBS three times, 100 µl of PBS containing 0.2% Triton X-100 was added, followed by a reaction at room temperature for 30 minutes. Further, after the product was washed with PBS three times, 100 µl of PBS containing 0.1% BSA was added, followed by a blocking reaction for 10 minutes. After the product was washed with PBS three times, 100 µl of an anti-M13 phage antibody (Amersham, mouse monoclonal antibody) diluted 1000-fold with 0.1% BSA/PBS, 100 µl of a fluorescein-labeled anti-mouse IgG antibody (Zymed Laboratories) diluted 2000-fold with 0.1% BSA/PBS, and 50 µl of an Alexa 488-labeled anti-fluorescein antibody (Molecular Probe) diluted 1000-fold with 0.1% BSA/PBS were sequentially reacted at room temperature for 30 minutes, for 30 minutes, and for 15 minutes, respectively. After each reaction, cells were washed with 150 µl of PBS three times. Cells after the final reaction were observed with a confocal laser fluorescent microscope (Olympus FV1000), and the numbers of luminescent spots derived from phages bound to an activated HUVEC and a non-activated HUVEC were compared, thereby, the cell binding property was assessed.

### <Results>

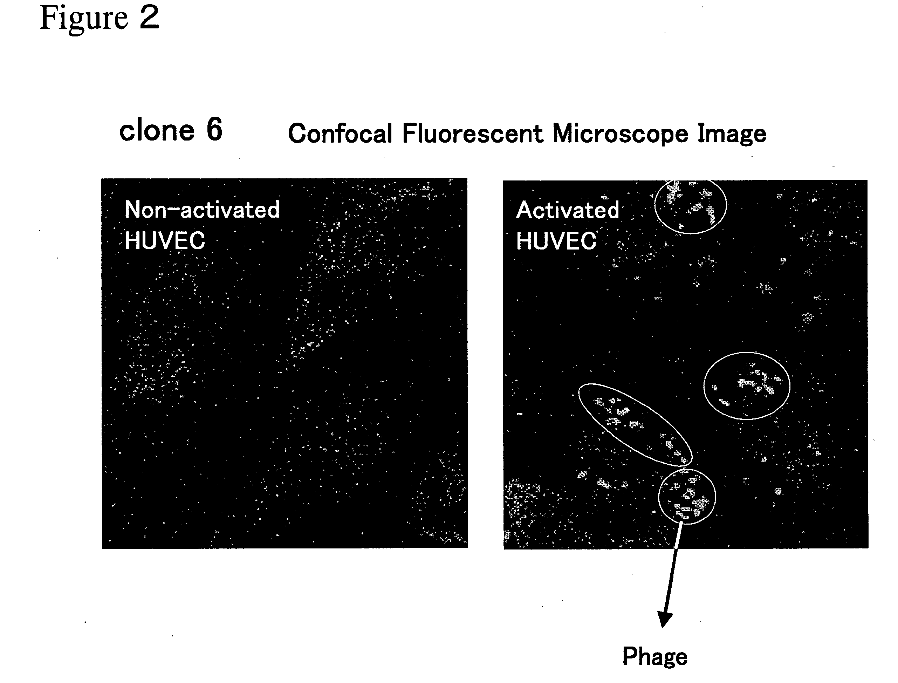

Phages bound to an activated HUVEC and a non-activated HUVEC were stained with a fluorescently labeled antibody, and observed with a confocal fluorescent microscope, thereby, the property of each phage clone to bind to each cell were scored, and the results are shown in Table 2. In addition, as representative results, a confocal fluorescent microscopic photograph of a clone 6 is shown in Fig. 2. In many clones, specific binding was observed in an activated HUVEC.

**[Table 2]**

| clone No. | | 3 | 5 | 6 | 11 | 12 | 20 | 23 | 25 | 28 | 29 | 34 |
|---|---|---|---|---|---|---|---|---|---|---|---|---|
| Binding potency to the cells | Non-activated HUVEC | - | - | - | + | - | - | - | - | - | ± | - |
| | Activated HUVEC | + | + | ++ | ++ | + | + | + | ± | ± | ± | - |

### Example 3 Synthesis of peptide, and preparation of polymer conjugate

A peptide having a nucleotide sequence determined in Example 1 was chemically synthesized by the Fmoc solid phase synthesis method. A fluorescent dye, fluorescein, was bound to an amino terminus of each peptide as an index for assessing binding and uptake into a cell. In addition, for the purpose of introducing a thiol group as a functional group for preparing the following polymer conjugate into a carboxyl terminus of a peptide, a sequence with a cysteine residue added thereto was synthesized.

In order to assess each peptide for the function to bind to, or to be taken into a cell as a drug carrier, a fluorescently labeled peptide-polymer conjugate was prepared by the following methods (1) and (2).

### (1) Preparation of peptide-multiarm PEG conjugate

PEG having four maleimide groups per molecule (Multiarm PEG, SUNBRIGHT PTE-200MA, manufactured by NOF Corporation) was dissolved in DMSO at the concentration of 2 mM, 20 µl of this solution, and an equivalent amount (20 µl) of a 10 mM fluorescein-labeled peptide solution in DMSO were mixed to react them at room temperature for 2 hours. After 60 µl of PBS was added to dilute the mixture to the total amount of 100 µl, desalting treatment was performed using a spin column (Bio-Rad, Micro Bio-Spin Column 30) equilibrated by washing with 500 µl of PBS three times in advance, and an unbound peptide was separated and removed to obtain a peptide-multiarm PEG conjugate.

### (2) Preparation of peptide-bovine serum albumin (BSA) conjugate

In 1 ml of a 50 mM sodium bicarbonate solution was dissolved 20 mg of BSA, to prepare a 0.3 mM solution. To the total amount (1 ml) of this solution was added 20 µl of a 250 mM solution (final concentration: 5 mM) of NHS-PEO12-maleimide (catalog No. 22112, manufactured by Pierce) which is a divalent crosslinking reagent having a PEG chain spacer, followed by a reaction at room temperature for 1 hour. After the reaction, 100 µl of the solution was desalted using a spin column (Bio-Rad, Micro Bio-Spin Column 30) equilibrated by washing with 500 µl of a 0.1 M sodium phosphate buffer (pH 7.0) three times in advance. An unreacted crosslinking reagent was separated and removed. After desalting, 50 µl of the solution was mixed with an equivalent amount of a 0.1 M sodium phosphate buffer (pH 7.0) to dilute, and to 100 µl of the resulting solution was added 10 µl of a fluorescein-labeled peptide solution in DMSO, followed by a reaction at room temperature for 1 hour. The total amount of the solution after the reaction was desalted using a spin column (Bio-Rad, Micro Bio-Spin Column 30) equilibrated by washing with 500 µl of PBS three times in advance, thereby, an unbound peptide was separated and removed, to obtain a peptide-BSA conjugate solution.

### Example 4 Uptake of fluorescently labeled peptide-PEG conjugate into endothelial cell (quantitative analysis)

### <Method>

A 96-well microplate (glass bottom plate, Nunc No. 164588) was treated with 100 µl of a 1% acidic collagen solution for 10 minutes in advance, and washed with 120 µl of PBS once, and 1 × 10⁵/ml of 100 µl of HUVEC cells were seeded, and cultured under the condition of 37°C and 5% CO₂ for 2 days. Upon use, cells were washed with 150 µl of a medium (EBM-2 medium containing a 2% fetal bovine serum (Cambrex Bio Science, containing a growth factor)) once, and 50 µl of the same medium, and 50 µl of a solution obtained by diluting a peptide-multiarm PEG conjugate (SEQ ID Nos. 12, 13, 16 (partial sequence of SEQ ID No. 14), and 77) prepared in Example 3 (1) with a medium to the peptide concentration of 20 µM were sequentially added, followed by culturing under the condition of 37°C and 5% CO₂ for 8 hours (the peptide concentration at reaction with cells was 10 µM). Cells after culturing were washed with 150 µl of a HBSS(+) buffer three times to remove an unbound peptide-multiarm PEG conjugate, and 100 µl of HBSS(+) containing 1% SDS to extract a peptide bound to, or taken into cells. The extract was transferred to another 96-well microplate (black plate), and fluorescence at an excitation wavelength of 490 nm and a detection wavelength of 530 nm was measured using a fluorophotometer. Letting the fluorescent intensity of each of the added peptide-multiarm PEG conjugate to be 100%, the amount (%) of uptake of the peptide into cells was calculated from the fluorescent amount recovered from cells.

### <Results>

The recovery rate of each peptide-multiarm PEG conjugate is shown in Table 3. As compared with the random sequence peptide (SEQ ID No. 77) which is a negative control, all of three kinds of peptides (SEQ ID Nos. 12, 13, and 16) of the present invention exhibited a high recovery rate.

**[Table 3]**

| Peptide | | Sequence | Recovery of Peptide-PEG conjugate (%) |
|---|---|---|---|
| Sequence-12 | Vascular endothelial cell-binding Peptide | Thr Leu Pro Ser Pro Leu Ala Leu Leu Thr Val His ( TLPSPLALLTVH ) | 0.211 |
| Sequence-13 | Vascular endothelial cell-binding Peptide | Ser Leu Thr Val Pro Phe Leu Pro Leu Tyr Val Pro ( SLTVPFLPLYVP ) | 0.122 |
| Sequence-16 | Vascular endothelial cell-binding Peptide | Gly Thr Gln Leu Phe Leu Ile His ( GTQLFLIH ) | 0.047 |
| Sequence-77 | Random sequence peptide ( Negative Control ) | Glu Tyr Asp Leu Ser Thr Ala Gly Gly Ala Ala Ala ( EYDLSTAGGAAA ) | 0.020 |

### Example 5 Uptake of fluorescently labeled peptide-BSA conjugate into endothelial cell (quantitative analysis)

### <Method>

On a 96-well microplate (Corning No. 3595) were seeded 100 µl of 1 × 10⁵/ml HUVEC cells, and cells were cultured under the condition of 37°C and 5% CO₂ for 2 days. Upon use, cells were washed with 150 µl of a medium (EBM-2 medium containing a 2% fetal bovine serum (Cambrex Bio Science, containing a growth factor)) once, and 50 µl of the same medium, and 50 µl of a solution obtained by diluting the peptide-BSA conjugate (SEQ ID Nos. 12, 13, 16, and 77) prepared in Example 3 (2) with a medium to the peptide concentration of 20 µm were sequentially added, followed by culturing under the condition of 37°C and 5% CO₂ for 8 hours (the peptide concentration at reaction with cells was 10 µM). Recovery of the peptide-BSA conjugate bound to, or taken into cells after the reaction, and fluorescence quantitation were performed in the same method as in Example 3.

### <Results>

The recovery rate of each peptide-BSA conjugate is shown in Table 4. As compared with a random sequence peptide (SEQ ID No. 77) as a negative control, all of three kinds of peptides (SEQ ID Nos. 12, 13, and 16) of the present invention exhibited a high recovery rate.

**[Table 4]**

| Peptide | | Sequence | Recovery of Peptide-BSA conjugate (%) |
|---|---|---|---|
| Sequence-12 | Vascular endothelial cell-binding Peptide | Thr Leu Pro Ser Pro Leu Ala Leu Leu Thr Val His ( TLPSPLALLTVH ) | 0.127 |
| Sequence-13 | Vascular endothelial cell-binding Peptide | Ser Leu Thr Val Pro Phe Leu Pro Leu Tyr Val Pro ( SLTVPFLPLYVP ) | 0.116 |
| Sequence-16 | Vascular endothelial cell-binding Peptide | Gly Thr Gln Leu Phe Leu Ile His ( GTQLFLIH ) | 0.227 |
| Sequence-77 | Random sequence peptide ( Negative Control ) | Glu Tyr Asp Leu Ser Thr Ala Gly Gly Ala Ala Ala ( EYDLSTAGGAAA ) | 0.029 |

### Example 6 Uptake of fluorescently labeled peptide-BSA conjugate into an endothelial cell (HUVEC) and a HeLa cell (image analysis)

### <Method>

A 96-well microplate (glass bottom plate, Nunc No. 164588) was treated with 100 µl of a 1% acidic collagen solution for 10 minutes in advance, and washed with 120 µl of PBS once, and 100 µl of 1 × 10⁵/ml of HUVEC cells were seeded, and cultured under the condition of 37°C and 5% CO₂ for 2 days. Upon use, cells were washed with 150 µl of a medium (EBM-2 medium containing a 2% fetal bovine serum (Cambrex Bio Science, containing a growth factor)) once, and 50 µl of the same medium, and 50 µl of a solution obtained by diluting the peptide-BSA conjugate (SEQ ID Nos. 12, 13, 16, and 77) prepared in Example 3 (2) with a medium to the peptide concentration of 20 µM were sequentially added, followed by culturing under the condition of 37°C and 5% CO₂ for 8 hours (the peptide concentration at reaction with cells was 10 µM). Cells after the reaction were observed using a confocal laser microscope (Olympus FV1000).

### <Results>

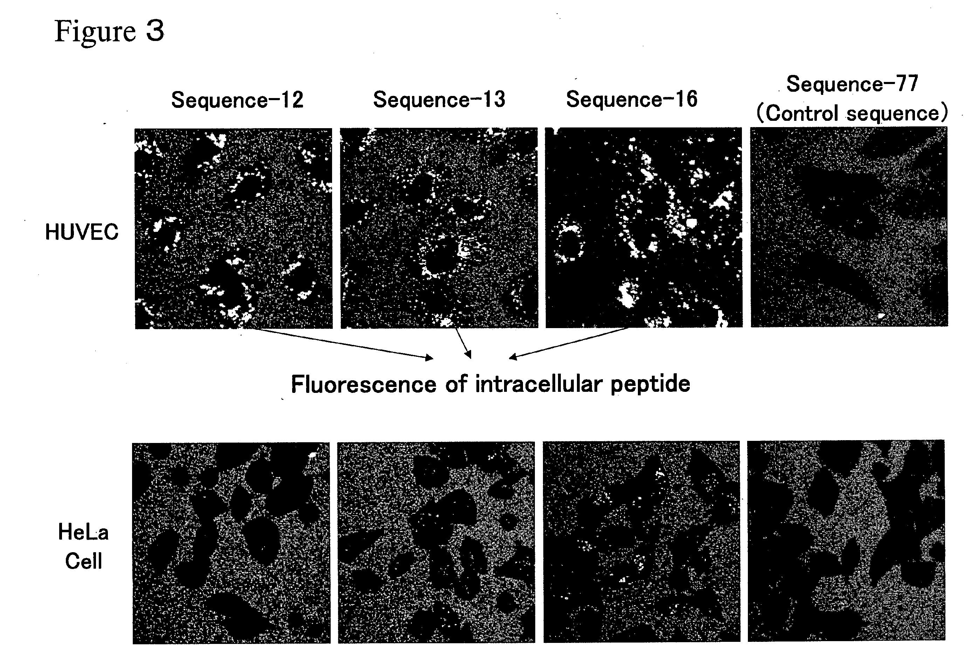

A confocal laser microscopic image of a cell reacted with each peptide-BSA conjugate is shown in Fig. 3. Regarding the HUVEC cells, many bright luminescent spots were observed in a cell for all of three kinds of peptides (SEQ ID Nos. 12, 13, and 16) of the present invention, and it was shown that the peptide was taken into a cell. Regarding the random sequence peptide (SEQ ID No. 77) as a negative control, no peptide in a cell was observed.

On the other hand, in the case of HeLa used as a comparative cell other than an endothelial cell, together with peptides of the present invention represented by SEQ ID Nos. 12, 13, and 16, an extremely small amount of peptides were observed in a cell as compared with uptake into a HUVEC, and it was shown that uptake is endothelial cell-specific.

### Example 7 Uptake of fluorescently labeled peptide-multiarm PEG conjugate into endothelial cell (image analysis scoring)

### <Method>

A 96-well microplate (glass bottom plate, Nunc No. 164588) was treated with 100 µl of a 1% acidic collagen solution for 10 minutes in advance, and washed with 120 µl of PBS once, and 100 µl of 1 × 10⁵/ml of HUVEC cells were seeded, and cultured under the condition of 37°C and 5% CO₂ for 2 days. Upon use, cells were washed with 150 µl of a medium (EBM-2 medium containing a 2% fetal bovine serum (Cambrex Bio Science, containing a growth factor)) once, and 50 µl of the same medium, and 50 µl of a solution obtained by diluting the multiarm PEG-peptide conjugate (a peptide of SEQ ID No. 12 and a partial peptide thereof (SEQ ID Nos. 17, 18, and 19), a peptide of SEQ ID No. 13 and a partial peptide thereof (SEQ ID Nos. 15, 20, and 21), and a random sequence peptide (SEQ ID No. 77)) prepared in Example 3 (1) with a medium to the peptide concentration of 32 µM were sequentially added, followed by culturing under the condition of 37°C and 5% CO₂ for 8 hours (the peptide concentration at reaction with cells was 16 µM). The cell after the reaction was observed using a confocal laser microscope (Olympus FV1000). The amount of fluorescent luminescent spots derived from the peptide recognized in a cell was scored as follows.

Score 0: No fluorescent luminescent spot in a cell is recognized.
Score 1: Faint fluorescent luminescent spots are recognized in a cell.
Score 2: Fluorescent luminescent spots clearer than in score 1 are recognized in a cell.
Score 3: Many luminescent spots further brighter than in score 2 are seen in a cell.

### <Results>

Results of scoring are shown in Table 5. For partial peptides (SEQ ID Nos. 17, 18, and 19) derived from the peptide of SEQ ID No. 12, intracellular fluorescence was recognized although weaker than in the original peptide of SEQ ID No. 12, and uptake into a cell was confirmed. In all of partial peptides (SEQ ID Nos. 15, 20, and 21) derived from the peptide of SEQ ID No. 13, fluorescence in a cell was recognized, and uptake into a cell was confirmed. Particularly, in a peptide (SEQ ID No. 15) corresponding to 5 to 12 amino acid residues of a 12-mer peptide of the peptide of SEQ ID No. 13, fluorescence equivalent to that of the peptide of SEQ ID No. 13 was recognized.

On the other hand, in the random sequence peptide (SEQ ID No. 77) of the negative control, no fluorescence in a cell was observed.

**[Table 5]**

| Peptie | Sequence | Intracellular Fluorescence (Score) |
|---|---|---|
| Sequence-12 | Thr Leu Pro Ser Pro Leu Ala Leu Leu Thr Val His | 3 |
| (Vascular endothelial cell-binding Peptide) | ( TLPSPLALLTVH ) | |
| Sequence-17 | Thr Leu Pro Ser Pro Leu Ala Leu | 1 |
| (Partial peptide derived from Sequence-12) | ( TLPSPLAL ) | |
| Sequence-18 | Pro Ser Pro Leu Ala Leu Leu Thr | 1 |
| (Partial peptide derived from Sequence-12) | ( PSPLALLT ) | |
| Sequence-19 | Pro Leu Ala Leu Leu Thr Val His | 1 |
| (Partial peptide derived from Sequence-12) | ( PLALLTVH ) | |
| Sequence-13 | Ser Leu Thr Val Pro Phe Leu Pro Leu Tyr Val Pro | 2 |
| (Vascular endothelial cell-binding Peptide) | ( SLTVPFLPLYVP ) | |
| Sequence-15 | Pro Phe Leu Pro Leu Tyr Val Pro | 2 |
| (Partial peptide derived from Sequence-13) | ( PFLPLYVP ) | |
| Sequence-20 | Ser Leu Thr Val Pro Phe Leu Pro | 1 |
| (Partial peptide derived from Sequence-13) | ( SLTVPFLP ) | |
| Sequence-21 | Thr Val Pro Phe Leu Pro Leu Tyr | 1 |
| (Partial peptide derived from Sequence-13) | ( TVPFLPLY ) | |
| Sequence-77 | Glu Tyr Asp Leu Ser Thr Ala Gly Gly Ala Ala Ala | 0 |
| (Random sequence) | ( EYDLSTAGGAAA ) | |

### Example 8 Assessment of accumulation of fluorescently labeled peptide-multiarm PEG conjugate in tumor

### <Method>

Mouse colon cancer cells (colon 26 cells) suspended in PBS were subcutaneously transplanted into a 8-week old BALB/c mouse (male) (purchased from Japan SLC) at 1.5 × 10⁶/mouse. After one month, in the state where a tumor diameter was about 1.5 cm, 200 uL of a PBS suspension of the multiarm PEG-peptide conjugate (SEQ ID Nos. 12 and 13) prepared in Example 3 (1) was administered into a tail vein. The mouse was euthanized 24 hours after administration, and a tumor tissue was isolated. The isolated tumor tissue was embedded using an OCT compound (Sakura Finetek) to prepare a frozen block. A frozen section of a tumor tissue was prepared from this frozen block using a cryostat (manufactured by Bright Instruments). The resulting tissue section was air-dried with the cold air for 1 hour. This tissue section was washed with PBS to remove the OCT compound, and immersed in acetone for 5 minutes to perform fixation treatment. After fixation treatment, PBS containing a 10% fetal bovine serum was reacted at room temperature for 30 minutes, thereby, blocking treatment was performed. Then, an antibody to a fluorescent dye, fluorescein, modified with a peptide (anti-fluorescein/Oregon Green rabbit IgG fraction Alexa Fluor 488 conjugate, Molecular Probes), and an antibody to a surface antigen CD31 known to be expressed in a vascular endothelial cell (Biotin-anti-mouse CD31, BD Pharmingen) were reacted at the dilution rate of 100-fold, and incubated at room temperature for 2 hours, thereby, a primary antibody reaction was performed. After completion of the reaction, washing was performed with PBS and, subsequently, a fluorescently labeled antibody to each primary antibody (Alexa Fluor 488 conjugate goat anti-rabbit IgG, Molecular Probes, and Streptavidin Alexa Fluor 594 conjugate, Invitrogen) were reacted, thereby, a secondary antibody reaction was performed. After completion of the secondary antibody reaction, washing with PBS was sufficiently performed, a sample was covered with a glass cover, observation was performed using a fluorescent microscope (IX-70, manufactured by Olympus), and accumulation of the peptide into a tumor tissue was assessed.

### <Results>

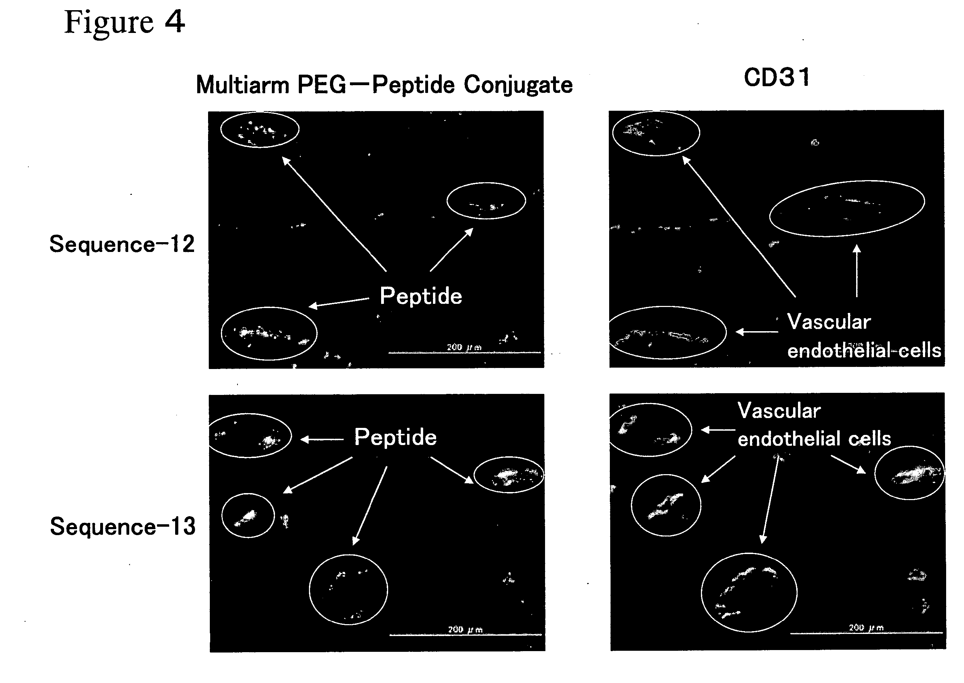

An image observed with a fluorescent microscope is shown in Fig. 4. As the result of observation, fluorescence derived from the peptide was observed in a tumor tissue, and it was shown that the intravenously administered fluorescently labeled peptide-PEG conjugate is accumulated in a tumor tissue. In addition, since a position of fluorescence derived from the peptide and a position stained with CD31 were consistent, it was shown that the fluorescently labeled peptide-multiarm PEG conjugate is localized in a blood vessel in a tumor tissue.

### Example 9 Preparation of peptide-modified liposome encapsulating doxorubicin

### <Method>

Hydrogenated soybean phosphatidyl choline (10 mg), cholesterol (3.4 mg), distearoylphosphatidylethanolamine-polyethylene glycol 5000 (3.5 mg), and distearoylphosphatidylethanolamine-polyethylene glycol 5000-maleimide (3.1 mg) were dissolved in chloroform, and a solvent in a flask was removed using an evaporator to form a thin film. To this was added 1.7 mL of a 250 mM aqueous ammonium sulfate solution, followed by heating at 65°C. The resulting vesicle was adjusted in a particle size with an extruder using, subsequently, porous membranes of the pore sizes of 1.0 µm, 0.4 µm, and 0.2 µm. Into the prepared liposome having the particle diameter of about 200 nm was encapsulated doxorubicin by a remote loading method. After unencapsulated doxorubicin was removed with a column, peptides (SEQ ID Nos. 12, 13, 76 (partial sequence of SEQ ID No. 7), and 78) were added, respectively, at the 1/5 amount based on maleimide, and a liposome into which various peptides are introduced was prepared. Unreacted maleimide was inactivated by adding the 3-fold amount of 2-mercaptoethanol.

### Example 10 Uptake of peptide-modified liposome encapsulating doxorubicin into HUVEC (quantification analysis)

### <Method>

Regarding a 50 to 60% confluent normal human umbilical vein endothelial cell cultured in a 24-well plate, a medium was exchanged, and a doxorubicin-encapsulated peptide-modified liposome solution was added in the half amount of a medium. After 6 hours, cells were washed with a phosphate buffer, treated with trypsin, and cells were collected by a centrifugation procedure. To a cell pellet was added 50 µL of 10% Triton X-100 to dissolve cells and the doxorubicin-encapsulated peptide-modified liposome, 350 µL of isopropanol with 10% 0.75 M HCl added thereto was added, and this was allowed to stand overnight at -20°C. After a centrifugation procedure, the supernatant was analyzed with an ODS column, and doxorubicin taken in the cell was quantitated.

### <Results>

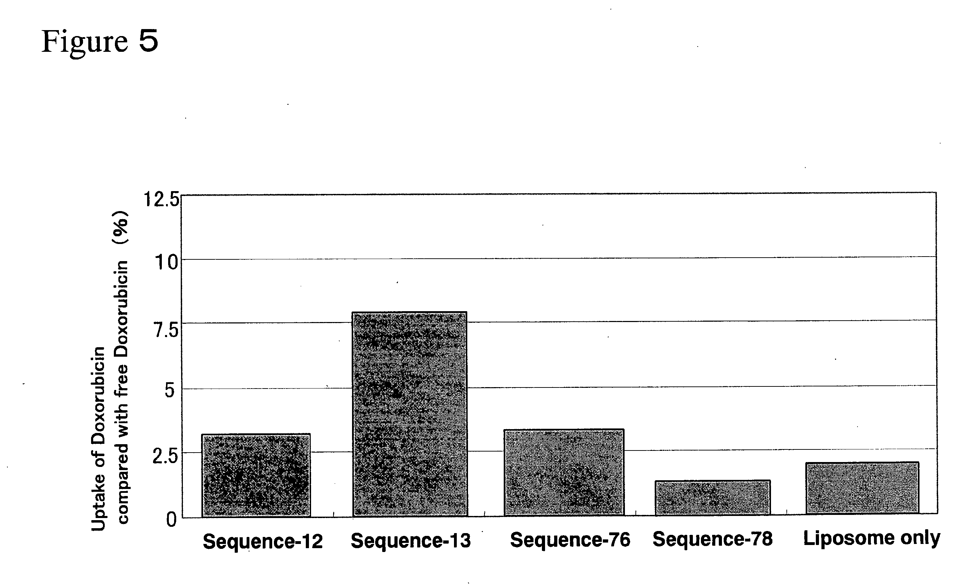

Results are shown in Fig. 5. In liposomes into which SEQ ID Nos. 12, 13, and 76 were introduced, the amount of uptake of doxorubicin was remarkably increased as compared with a liposome with the peptide of the random sequence (SEQ ID No. 78), and a liposome with no peptide introduced. Particularly, the amount of uptake of doxorubicin into a liposome with SEQ ID No. 13 introduced therein was higher as compared with that in introduction of SEQ ID Nos. 12 and 76, and was about 3-fold of that in no peptide introduction.

### Industrial Applicability

The peptide of the present invention can be expected to specifically accumulate in a new blood vessel present in a diseased tissue such as a solid tumor, and can be employed for treating and diagnosing various diseases accompanied with vascularization using this nature.

## Claims

1. A peptide of any one of the following (A), (B), and (C):
(A) a peptide comprising an amino acid sequence represented by any one of SEQ ID Nos. 1 to 76;
(B) a peptide having a sequence in which one or several amino acids are substituted, deleted, inserted or added in an amino acid sequence represented by any one of SEQ ID Nos. 1 to 76, and having an ability to bind to, or to be taken into an activated vascular endothelial cell; and
(C) a peptide containing the peptide of (A) or (B) as a partial sequence, and having an ability to bind to, or to be taken into an activated vascular endothelial cell.

2. A nucleic acid encoding the peptide as defined in claim 1.

3. A peptide binding body wherein the peptide as defined in claim 1 is bound to a hydrophilic polymer.

4. A pharmaceutical composition comprising the peptide as defined in claim 1.

5. A method of accumulating a drug in a new blood vessel, comprising using the peptide as defined in claim 1.


SEQUENCE LISTING

<110>  Toray Industries, Inc.

<120>  Vascular endothelial cell binding peptides

<130>  660M03001

<160>  78

<170>  PatentIn version 3.1

<210>  1
<211>  15
<212>  PRT
<213>  Artificial

<220>
<223>  Designed peptide acting as a vascular endothelial cell binding pe
       ptide

<400>  1

Asp Arg Arg Val Ser Leu Arg Ile Pro Phe Ser Ile Leu His Arg
1               5                   10                  15


<210>  2
<211>  15
<212>  PRT
<213>  Artificial

<220>
<223>  Designed peptide acting as a vascular endothelial cell binding pe
       ptide

<400>  2

Ser Ser Tyr Lys Trp Leu Leu Ala Asp Tyr Pro Gln Arg Leu Leu
1               5                   10                  15


<210>  3
<211>  15
<212>  PRT
<213>  Artificial

<220>
<223>  Designed peptide acting as a vascular endothelial cell binding pe
       ptide

<400>  3

Ser His Val Ala Ser Leu Leu Pro Ala Leu Ala Lys Gly Gly Arg
1               5                   10                  15


<210>  4
<211>  15
<212>  PRT
<213>  Artificial

<220>
<223>  Designed peptide acting as a vascular endothelial cell binding pe
      ptide

<400>  4

Asn Arg Gly Tyr Ser Ser Phe Gln Thr Phe Gly His Leu Leu Leu
1               5                   10                  15


<210>  5
<211>  15
<212>  PRT
<213>  Artificial

<220>
<223>  Designed peptide acting as a vascular endothelial cell binding pe
      ptide

<400>  5

Arg Trp Leu Pro Val Leu Ser Leu Lys Tyr Val Lys Trp Glu His
1               5                   10                  15


<210>  6
<211>  15
<212>  PRT
<213>  Artificial

<220>
<223>  Designed peptide acting as a vascular endothelial cell binding pe
      ptide

<400>  6

Arg Leu Trp Met Leu Arg Ala Asp Phe Val Ser Ser Arg Thr Asp
1               5                   10                  15


<210>  7
<211>  15
<212>  PRT
<213>  Artificial

<220>
<223>  Designed peptide acting as a vascular endothelial cell binding pe
      ptide

<400>  7

Leu Gln Phe Pro Leu Val Ile Pro Ile Leu Ser Ile Tyr Thr Ala
1               5                   10                  15


<210>  8
<211>  15
<212>  PRT
<213>  Artificial

<220>
<223>  Designed peptide acting as a vascular endothelial cell binding pe
      ptide

<400>  8

Lys Lys Asp Leu Ile Asn Leu Ala Val Gly Phe Ile Asp Ser Phe
1               5                   10                  15


<210>  9
<211>  15
<212>  PRT
<213>  Artificial

<220>
<223>  Designed peptide acting as a vascular endothelial cell binding pe
      ptide

<400>  9

Ile Thr Leu Val His Pro Gly Ala Tyr Phe His Phe Leu Leu Asp
1               5                   10                  15


<210>  10
<211>  15
<212>  PRT
<213>  Artificial

<220>
<223>  Designed peptide acting as a vascular endothelial cell binding pe
      ptide

<400>  10

Tyr Trp Phe Ser Ile Leu Gly Asp Leu Leu Pro Glu Val Ala Asn
1               5                   10                  15


<210>  11
<211>  15
<212>  PRT
<213>  Artificial

<220>
<223>  Designed peptide acting as a vascular endothelial cell binding pe
      ptide

<400>  11

Gly Met Arg Pro Arg Leu Ala Ser Asn Ser Gly Met Ser Asn Leu
1               5                   10                  15


<210>  12
<211>  12
<212>  PRT
<213>  Artificial

<220>

<223>  Designed peptide acting as a vascular endothelial cell binding pe
       ptide

<400>  12

Thr Leu Pro Ser Pro Leu Ala Leu Leu Thr Val His
1               5                   10


<210>  13
<211>  12
<212>  PRT
<213>  Artificial

<220>
<223>  Designed peptide acting as a vascular endothelial cell binding pe
       ptide

<400>  13

Ser Leu Thr Val Pro Phe Leu Pro Leu Tyr Val Pro
1               5                   10


<210>  14
<211>  15
<212>  PRT
<213>  Artificial

<220>
<223>  Designed peptide acting as a vascular endothelial cell binding pe
       ptide

<400>  14

Ala Lys Val Gly Thr Gln Leu Phe Leu Ile His Ala Ser Ile Phe
1               5                   10                  15


<210>  15
<211>  8
<212>  PRT
<213>  Artificial

<220>
<223>  Designed peptide acting as a vascular endothelial cell binding pe
       ptide

<400>  15

Pro Phe Leu Pro Leu Tyr Val Pro
1               5


<210>  16
<211>  8
<212>  PRT
<213>  Artificial

<220>
<223>  Designed peptide acting as a vascular endothelial cell binding pe

ptide

<400>  16

Gly Thr Gln Leu Phe Leu Ile His
1               5


<210>  17
<211>  8
<212>  PRT
<213>  Artificial

<220>
<223>  Designed peptide acting as a vascular endothelial cell binding pe
       ptide

<400>  17

Thr Leu Pro Ser Pro Leu Ala Leu
1               5


<210>  18
<211>  8
<212>  PRT
<213>  Artificial

<220>
<223>  Designed peptide acting as a vascular endothelial cell binding pe
       ptide

<400>  18

Pro Ser Pro Leu Ala Leu Leu Thr
1               5


<210>  19
<211>  8
<212>  PRT
<213>  Artificial

<220>
<223>  Designed peptide acting as a vascular endothelial cell binding pe
       ptide

<400>  19

Pro Leu Ala Leu Leu Thr Val His
1               5


<210>  20
<211>  8
<212>  PRT
<213>  Artificial

<220>
<223>  Designed peptide acting as a vascular endothelial cell binding pe
       ptide

<400>  20

Ser Leu Thr Val Pro Phe Leu Pro
1               5


<210>  21
<211>  8
<212>  PRT
<213>  Artificial

<220>
<223>  Designed peptide acting as a vascular endothelial cell binding pe
       ptide

<400>  21

Thr Val Pro Phe Leu Pro Leu Tyr
1               5


<210>  22
<211>  15
<212>  PRT
<213>  Artificial

<220>
<223>  Designed peptide acting as a vascular endothelial cell binding pe
       ptide

<400>  22

Ser Tyr Leu Asn Ser Lys Leu Leu Pro Pro Ser Ala Leu Thr Gly
1               5                   10                  15


<210>  23
<211>  15
<212>  PRT
<213>  Artificial

<220>
<223>  Designed peptide acting as a vascular endothelial cell binding pe
       ptide

<400>  23

Ser Gly Ile Ser Trp Pro Leu Glu Ala Leu Ala Leu Trp Leu Leu
1               5                   10                  15


<210>  24
<211>  15
<212>  PRT
<213>  Artificial

<220>
<223>  Designed peptide acting as a vascular endothelial cell binding pe
       ptide

<400>  24

Gly Ile Ala Trp Thr Asn Arg Ile Val Ser Asp Ala Val Glu Pro
1               5                   10                  15


<210>  25
<211>  15
<212>  PRT
<213>  Artificial

<220>
<223>  Designed peptide acting as a vascular endothelial cell binding pe
       ptide

<400>  25

Ala Asn Asn Pro Trp Gln Glu Met Ile Ser Tyr Glu Lys Ile His
1               5                   10                  15


<210>  26
<211>  15
<212>  PRT
<213>  Artificial

<220>
<223>  Designed peptide acting as a vascular endothelial cell binding pe
       ptide

<400>  26

Ser Arg Val Pro Gly Met Tyr Asn Asp Gln Arg Ala Thr Phe Phe
1               5                   10                  15


<210>  27
<211>  15
<212>  PRT
<213>  Artificial

<220>
<223>  Designed peptide acting as a vascular endothelial cell binding pe
       ptide

<400>  27

Cys Tyr Leu Thr Met Thr Ala Val Ser Arg Ser Gln Tyr Ser Leu
1               5                   10                  15


<210>  28
<211>  15
<212>  PRT
<213>  Artificial

<220>
<223>  Designed peptide acting as a vascular endothelial cell binding pe
       ptide

<400>  28

Thr Thr Arg Val Trp Leu Asp Gln Met Val Glu Pro Gly Asn Glu
1               5                   10                  15

<210>  29
<211>  15
<212>  PRT
<213>  Artificial

<220>
<223>  Designed peptide acting as a vascular endothelial cell binding pe
       ptide

<400>  29

Asn Leu His Trp Thr Leu Thr Phe Ser Lys Val Pro Thr Gly Glu
1               5                   10                  15

<210>  30
<211>  15
<212>  PRT
<213>  Artificial

<220>
<223>  Designed peptide acting as a vascular endothelial cell binding pe
       ptide

<400>  30

Gly Val Gly Gln Leu Ala Met Thr Leu Ser Gly Arg Gly Ser His
1               5                   10                  15

<210>  31
<211>  15
<212>  PRT
<213>  Artificial

<220>
<223>  Designed peptide acting as a vascular endothelial cell binding pe
       ptide

<400>  31

Ser Arg Thr Pro Asn Asn Ile Met Ala Val Asn Ser His Ser Arg
1               5                   10                  15

<210>  32
<211>  15
<212>  PRT
<213>  Artificial

<220>
<223>  Designed peptide acting as a vascular endothelial cell binding pe
       ptide

<400>  32

Ser Met Val Met Asp Leu Arg Gly Lys Phe Pro Ser Val Arg Val
1               5                   10                  15

<210>  33
<211>  15
<212>  PRT
<213>  Artificial

<220>
<223>  Designed peptide acting as a vascular endothelial cell binding pe
       ptide

<400>  33

Ser Lys Ala Pro Ser Trp Asp Leu Pro Lys Thr Gly Gly Glu Ile
1               5                   10                  15

<210>  34
<211>  15
<212>  PRT
<213>  Artificial

<220>
<223>  Designed peptide acting as a vascular endothelial cell binding pe
       ptide

<400>  34

Ala Arg Arg Pro Ala Val Lys Ala Val Thr Thr Asp Ser Tyr Gly
1               5                   10                  15

<210>  35
<211>  15
<212>  PRT
<213>  Artificial

<220>
<223>  Designed peptide acting as a vascular endothelial cell binding pe
       ptide

<400>  35

Ser Arg Met Pro Met Gly Val His Asp Thr Thr Ala Leu Lys Trp
1               5                   10                  15

<210>  36
<211>  15
<212>  PRT
<213>  Artificial

<220>
<223>  Designed peptide acting as a vascular endothelial cell binding pe
       ptide

<400>  36

Gln Ser Leu Ile Tyr Ser Ser Leu Phe Pro Arg Arg Ser Trp Phe

1               5                   10                  15


<210>  37
<211>  15
<212>  PRT
<213>  Artificial

<220>
<223>  Designed peptide acting as a vascular endothelial cell binding pe
       ptide

<400>  37

Met Thr Leu Ser Met Ala Arg Thr Ala Arg Asp Ile Gly Thr Gly
1               5                   10                  15


<210>  38
<211>  12
<212>  PRT
<213>  Artificial

<220>
<223>  Designed peptide acting as a vascular endothelial cell binding pe
       ptide

<400>  38

Ser Pro Arg Pro Leu Pro Ile Ile Thr Pro Phe Pro
1               5                   10


<210>  39
<211>  12
<212>  PRT
<213>  Artificial

<220>
<223>  Designed peptide acting as a vascular endothelial cell binding pe
       ptide

<400>  39

His Val Thr Ser Tyr Asp Ala Trp Ala Pro Asp Pro
1               5                   10


<210>  40
<211>  12
<212>  PRT
<213>  Artificial

<220>
<223>  Designed peptide acting as a vascular endothelial cell binding pe
       ptide

<400>  40

Asp Tyr Pro Lys Ala Pro Gly Ser His Pro Arg Thr
1               5                   10

<210>  41
<211>  12
<212>  PRT
<213>  Artificial

<220>
<223>  Designed peptide acting as a vascular endothelial cell binding pe
       ptide

<400>  41

Thr Ser Lys Phe Pro Ser Thr Asp Leu Ala Arg Leu
1               5                   10


<210>  42
<211>  12
<212>  PRT
<213>  Artificial

<220>
<223>  Designed peptide acting as a vascular endothelial cell binding pe
       ptide

<400>  42

Val Gly Asn Ser Asp Thr Thr Gly Thr Ser Arg Val
1               5                   10


<210>  43
<211>  12
<212>  PRT
<213>  Artificial

<220>
<223>  Designed peptide acting as a vascular endothelial cell binding pe
       ptide

<400>  43

Ala Thr Trp Ser His His Leu Ser Ser Ala Gly Leu
1               5                   10


<210>  44
<211>  12
<212>  PRT
<213>  Artificial

<220>
<223>  Designed peptide acting as a vascular endothelial cell binding pe
       ptide

<400>  44

Met Gly Tyr Asp Phe Thr Arg Ser Pro Leu Thr Trp
1               5                   10

<210>  45
<211>  12
<212>  PRT
<213>  Artificial

<220>
<223>  Designed peptide acting as a vascular endothelial cell binding pe
       ptide

<400>  45

Met Ala Asn Leu Thr Gly Ser Gly Thr His Asn Leu
1               5                   10


<210>  46
<211>  12
<212>  PRT
<213>  Artificial

<220>
<223>  Designed peptide acting as a vascular endothelial cell binding pe
       ptide

<400>  46

His Leu Ser Ser Arg Asp Leu Ser Leu Thr Ser Leu
1               5                   10


<210>  47
<211>  12
<212>  PRT
<213>  Artificial

<220>
<223>  Designed peptide acting as a vascular endothelial cell binding pe
       ptide

<400>  47

Trp Gly Ile Ser Ile Thr Asn Pro Ala Ala Leu Ser
1               5                   10


<210>  48
<211>  12
<212>  PRT
<213>  Artificial

<220>
<223>  Designed peptide acting as a vascular endothelial cell binding pe
       ptide

<400>  48

Asp Val Asn Lys Leu Leu Leu Arg Leu Pro Val Thr
1               5                   10

<210>  49
<211>  12
<212>  PRT
<213>  Artificial

<220>
<223>  Designed peptide acting as a vascular endothelial cell binding pe
       ptide

<400>  49

Ala Ile Pro Ile Gly Thr Leu Pro Lys Ile His Leu
1               5                   10


<210>  50
<211>  12
<212>  PRT
<213>  Artificial

<220>
<223>  Designed peptide acting as a vascular endothelial cell binding pe
       ptide

<400>  50

Tyr Pro Gln Ala Ser Leu Ser Thr Phe Lys Pro Leu
1               5                   10


<210>  51
<211>  12
<212>  PRT
<213>  Artificial

<220>
<223>  Designed peptide acting as a vascular endothelial cell binding pe
       ptide

<400>  51

Asn Val Phe Arg His Ala Ile Glu Gln Arg Thr Pro
1               5                   10


<210>  52
<211>  12
<212>  PRT
<213>  Artificial

<220>
<223>  Designed peptide acting as a vascular endothelial cell binding pe
       ptide

<400>  52

Ser Phe Tyr Asn Ala Asp Ser Ser Val Ser Arg Leu
1               5                   10


<210>  53

<211>  12
<212>  PRT
<213>  Artificial

<220>
<223>  Designed peptide acting as a vascular endothelial cell binding pe
      ptide

<400>  53

Trp Ser Pro Ser Gln Phe Lys Leu Asp Met Pro His
1               5                   10


<210>  54
<211>  12
<212>  PRT
<213>  Artificial

<220>
<223>  Designed peptide acting as a vascular endothelial cell binding pe
      ptide

<400>  54

Asn Ile Ala Lys Thr Ser Asn Ser Ser His Leu Pro
1               5                   10


<210>  55
<211>  12
<212>  PRT
<213>  Artificial

<220>
<223>  Designed peptide acting as a vascular endothelial cell binding pe
      ptide

<400>  55

Gly Pro Met Leu Asp Met Ala Leu Gly Pro Ala Pro
1               5                   10


<210>  56
<211>  12
<212>  PRT
<213>  Artificial

<220>
<223>  Designed peptide acting as a vascular endothelial cell binding pe
      ptide

<400>  56

Lys Ser Phe Leu Tyr Thr Pro Ala Thr Val Thr His
1               5                   10


<210>  57
<211>  12

<212>  PRT
<213>  Artificial

<220>
<223>  Designed peptide acting as a vascular endothelial cell binding pe
       ptide

<400>  57

Tyr Ala Thr Val His Thr Pro Leu Ala Trp Leu Pro
1               5                   10


<210>  58
<211>  12
<212>  PRT
<213>  Artificial

<220>
<223>  Designed peptide acting as a vascular endothelial cell binding pe
       ptide

<400>  58

Trp Met Pro Thr Lys Pro His Leu His Asn Leu Gly
1               5                   10


<210>  59
<211>  12
<212>  PRT
<213>  Artificial

<220>
<223>  Designed peptide acting as a vascular endothelial cell binding pe
       ptide

<400>  59

Glu Asn Pro Ser Pro Tyr Ile Pro Ile Pro Leu Thr
1               5                   10


<210>  60
<211>  12
<212>  PRT
<213>  Artificial

<220>
<223>  Designed peptide acting as a vascular endothelial cell binding pe
       ptide

<400>  60

Glu Val Pro Leu Thr Gln Phe Leu Trp His Gln Leu
1               5                   10


<210>  61
<211>  12
<212>  PRT

<213>  Artificial

<220>
<223>  Designed peptide acting as a vascular endothelial cell binding pe
      ptide

<400>  61

Met Ser Lys Asp Gln Pro Ser Phe Phe Arg Thr Ser
1               5                   10


<210>  62
<211>  12
<212>  PRT
<213>  Artificial

<220>
<223>  Designed peptide acting as a vascular endothelial cell binding pe
      ptide

<400>  62

His Ala Ala Tyr Leu Val Glu Trp Pro Gly Ala Gly
1               5                   10


<210>  63
<211>  12
<212>  PRT
<213>  Artificial

<220>
<223>  Designed peptide acting as a vascular endothelial cell binding pe
      ptide

<400>  63

Leu Pro Val Gln Leu Pro Val Asn Ile Ser Pro Arg
1               5                   10


<210>  64
<211>  12
<212>  PRT
<213>  Artificial

<220>
<223>  Designed peptide acting as a vascular endothelial cell binding pe
      ptide

<400>  64

Gln Leu Leu Asn Ala Leu Pro Phe Arg Leu Ala Tyr
1               5                   10


<210>  65
<211>  12
<212>  PRT
<213>  Artificial

<220>
<223>  Designed peptide acting as a vascular endothelial cell binding pe
       ptide

<400>  65

Ala Met Gly His Gly Leu Ala Lys Val Thr Thr Arg
1               5                   10


<210>  66
<211>  12
<212>  PRT
<213>  Artificial

<220>
<223>  Designed peptide acting as a vascular endothelial cell binding pe
       ptide

<400>  66

Thr Asn Ser His Ala Val Pro Leu Leu Pro Leu Leu
1               5                   10


<210>  67
<211>  12
<212>  PRT
<213>  Artificial

<220>
<223>  Designed peptide acting as a vascular endothelial cell binding pe
       ptide

<400>  67

Asp Ser Thr Met Met Pro Thr Val Leu Ser Thr Leu
1               5                   10


<210>  68
<211>  12
<212>  PRT
<213>  Artificial

<220>
<223>  Designed peptide acting as a vascular endothelial cell binding pe
       ptide

<400>  68

Val Ser Gly Ser Glu Thr His Thr Met Pro Leu Ala
1               5                   10


<210>  69
<211>  12
<212>  PRT
<213>  Artificial

<220>
<223>  Designed peptide acting as a vascular endothelial cell binding pe
      ptide

<400>  69

His Lys Leu Ala Thr Ser Pro Trp Trp Pro Pro Ile
1               5                   10


<210>  70
<211>  12
<212>  PRT
<213>  Artificial

<220>
<223>  Designed peptide acting as a vascular endothelial cell binding pe
      ptide

<400>  70

Ser Thr His His Arg His Tyr His Asp Thr Leu Ala
1               5                   10


<210>  71
<211>  12
<212>  PRT
<213>  Artificial

<220>
<223>  Designed peptide acting as a vascular endothelial cell binding pe
      ptide

<400>  71

Phe Ala Ser Ser His Lys Gln Ile Pro Leu Gln Leu
1               5                   10


<210>  72
<211>  12
<212>  PRT
<213>  Artificial

<220>
<223>  Designed peptide acting as a vascular endothelial cell binding pe
      ptide

<400>  72

Asp Ser Leu Tyr Thr Leu Phe Pro Arg Ser Ala Phe
1               5                   10


<210>  73
<211>  12
<212>  PRT
<213>  Artificial

<220>

<223>  Designed peptide acting as a vascular endothelial cell binding pe
      ptide

<400>  73

His Asn Pro Ile Pro Tyr Thr Ala Pro Leu Leu Phe
1               5                   10


<210>  74
<211>  12
<212>  PRT
<213>  Artificial

<220>
<223>  Designed peptide acting as a vascular endothelial cell binding pe
      ptide

<400>  74

Met Ile Asn Thr Lys Ile Pro Pro Phe Thr Arg Trp
1               5                   10


<210>  75
<211>  12
<212>  PRT
<213>  Artificial

<220>
<223>  Designed peptide acting as a vascular endothelial cell binding pe
      ptide

<400>  75

His Ala Lys Ser Gln Pro Ile Asn Ser Phe Ser Met
1               5                   10


<210>  76
<211>  10
<212>  PRT
<213>  Artificial

<220>
<223>  Designed peptide acting as a vascular endothelial cell binding pe
      ptide

<400>  76

Val Ile Pro Ile Leu Ser Ile Tyr Thr Ala
1               5                   10


<210>  77
<211>  12
<212>  PRT
<213>  Artificial

<220>
<223>  control sequence

<400>  77

Glu Tyr Asp Leu Ser Thr Ala Gly Gly Ala Ala Ala
1               5                   10

<210>  78
<211>  19
<212>  PRT
<213>  Artificial

<220>
<223>  control sequence

<400>  78

Gly Leu Asp Lys Ser Ser Tyr Arg Ile Asp Thr Phe Ala Ala His Glu
1               5                   10                  15

Val Ala Gly

SEQUENCE LISTING

<110> Toray Industries, Inc.

<120> Vascular endothelial cell binding peptides

<130> 660M03001

<160> 78

<170> PatentIn version 3.1

<210> 1
<211> 15
<212> PRT
<213> Artificial

<220>
<223> Designed peptide acting as a vascular endothelial cell binding pe
ptide

<400> 1

Asp Arg Arg Val Ser Leu Arg Ile Pro Phe Ser Ile Leu His Arg
1               5                   10                  15


<210> 2
<211> 15
<212> PRT
<213> Artificial

<220>
<223> Designed peptide acting as a vascular endothelial cell binding pe
ptide

<400> 2

Ser Ser Tyr Lys Trp Leu Leu Ala Asp Tyr Pro Gln Arg Leu Leu
1               5                   10                  15


<210> 3
<211> 15
<212> PRT
<213> Artificial

<220>
<223> Designed peptide acting as a vascular endothelial cell binding pe
ptide

<400> 3

Ser His Val Ala Ser Leu Leu Pro Ala Leu Ala Lys Gly Gly Arg
1               5                   10                  15


<210> 4
<211> 15
<212> PRT
<213> Artificial

<220>
<223> Designed peptide acting as a vascular endothelial cell binding pe
ptide

<400> 4

Asn Arg Gly Tyr Ser Ser Phe Gln Thr Phe Gly His Leu Leu Leu

1                   5                   10                  15

<210>  5
<211>  15
<212>  PRT
<213>  Artificial

<220>
<223>  Designed peptide acting as a vascular endothelial cell binding pe
       ptide

<400>  5

Arg Trp Leu Pro Val Leu Ser Leu Lys Tyr Val Lys Trp Glu His
1               5                   10                  15

<210>  6
<211>  15
<212>  PRT
<213>  Artificial

<220>
<223>  Designed peptide acting as a vascular endothelial cell binding pe
       ptide

<400>  6

Arg Leu Trp Met Leu Arg Ala Asp Phe Val Ser Ser Arg Thr Asp
1               5                   10                  15

<210>  7
<211>  15
<212>  PRT
<213>  Artificial

<220>
<223>  Designed peptide acting as a vascular endothelial cell binding pe
       ptide

<400>  7

Leu Gln Phe Pro Leu Val Ile Pro Ile Leu Ser Ile Tyr Thr Ala
1               5                   10                  15

<210>  8
<211>  15
<212>  PRT
<213>  Artificial

<220>
<223>  Designed peptide acting as a vascular endothelial cell binding pe
       ptide

<400>  8

Lys Lys Asp Leu Ile Asn Leu Ala Val Gly Phe Ile Asp Ser Phe
1               5                   10                  15

<210>  9
<211>  15
<212>  PRT
<213>  Artificial

<220>

<223>  Designed peptide acting as a vascular endothelial cell binding pe
      ptide

<400>  9

Ile Thr Leu Val His Pro Gly Ala Tyr Phe His Phe Leu Leu Asp
1               5                   10                  15


<210>  10
<211>  15
<212>  PRT
<213>  Artificial

<220>
<223>  Designed peptide acting as a vascular endothelial cell binding pe
      ptide

<400>  10

Tyr Trp Phe Ser Ile Leu Gly Asp Leu Leu Pro Glu Val Ala Asn
1               5                   10                  15


<210>  11
<211>  15
<212>  PRT
<213>  Artificial

<220>
<223>  Designed peptide acting as a vascular endothelial cell binding pe
      ptide

<400>  11

Gly Met Arg Pro Arg Leu Ala Ser Asn Ser Gly Met Ser Asn Leu
1               5                   10                  15


<210>  12
<211>  12
<212>  PRT
<213>  Artificial

<220>
<223>  Designed peptide acting as a vascular endothelial cell binding pe
      ptide

<400>  12

Thr Leu Pro Ser Pro Leu Ala Leu Leu Thr Val His
1               5                   10


<210>  13
<211>  12
<212>  PRT
<213>  Artificial

<220>
<223>  Designed peptide acting as a vascular endothelial cell binding pe
      ptide

<400>  13

Ser Leu Thr Val Pro Phe Leu Pro Leu Tyr Val Pro
1               5                   10

<210>  14
<211>  15
<212>  PRT
<213>  Artificial

<220>
<223>  Designed peptide acting as a vascular endothelial cell binding pe
       ptide

<400>  14

Ala Lys Val Gly Thr Gln Leu Phe Leu Ile His Ala Ser Ile Phe
1               5                   10                  15


<210>  15
<211>  8
<212>  PRT
<213>  Artificial

<220>
<223>  Designed peptide acting as a vascular endothelial cell binding pe
       ptide

<400>  15

Pro Phe Leu Pro Leu Tyr Val Pro
1               5


<210>  16
<211>  8
<212>  PRT
<213>  Artificial

<220>
<223>  Designed peptide acting as a vascular endothelial cell binding pe
       ptide

<400>  16

Gly Thr Gln Leu Phe Leu Ile His
1               5


<210>  17
<211>  8
<212>  PRT
<213>  Artificial

<220>
<223>  Designed peptide acting as a vascular endothelial cell binding pe
       ptide

<400>  17

Thr Leu Pro Ser Pro Leu Ala Leu
1               5


<210>  18
<211>  8
<212>  PRT
<213>  Artificial

<220>
<223>  Designed peptide acting as a vascular endothelial cell binding pe
       ptide

<400>  18

Pro Ser Pro Leu Ala Leu Leu Thr
1               5


<210>  19
<211>  8
<212>  PRT
<213>  Artificial

<220>
<223>  Designed peptide acting as a vascular endothelial cell binding pe
       ptide

<400>  19

Pro Leu Ala Leu Leu Thr Val His
1               5


<210>  20
<211>  8
<212>  PRT
<213>  Artificial

<220>
<223>  Designed peptide acting as a vascular endothelial cell binding pe
       ptide

<400>  20

Ser Leu Thr Val Pro Phe Leu Pro
1               5


<210>  21
<211>  8
<212>  PRT
<213>  Artificial

<220>
<223>  Designed peptide acting as a vascular endothelial cell binding pe
       ptide

<400>  21

Thr Val Pro Phe Leu Pro Leu Tyr
1               5


<210>  22
<211>  15
<212>  PRT
<213>  Artificial

<220>
<223>  Designed peptide acting as a vascular endothelial cell binding pe
       ptide

<400>  22

Ser Tyr Leu Asn Ser Lys Leu Leu Pro Pro Ser Ala Leu Thr Gly
1               5                   10                  15


<210>  23
<211>  15
<212>  PRT

<213>  Artificial

<220>
<223>  Designed peptide acting as a vascular endothelial cell binding pe
      ptide

<400>  23

Ser Gly Ile Ser Trp Pro Leu Glu Ala Leu Ala Leu Trp Leu Leu
1               5                   10                  15


<210>  24
<211>  15
<212>  PRT
<213>  Artificial

<220>
<223>  Designed peptide acting as a vascular endothelial cell binding pe
      ptide

<400>  24

Gly Ile Ala Trp Thr Asn Arg Ile Val Ser Asp Ala Val Glu Pro
1               5                   10                  15


<210>  25
<211>  15
<212>  PRT
<213>  Artificial

<220>
<223>  Designed peptide acting as a vascular endothelial cell binding pe
      ptide

<400>  25

Ala Asn Asn Pro Trp Gln Glu Met Ile Ser Tyr Glu Lys Ile His
1               5                   10                  15


<210>  26
<211>  15
<212>  PRT
<213>  Artificial

<220>
<223>  Designed peptide acting as a vascular endothelial cell binding pe
      ptide

<400>  26

Ser Arg Val Pro Gly Met Tyr Asn Asp Gln Arg Ala Thr Phe Phe
1               5                   10                  15


<210>  27
<211>  15
<212>  PRT
<213>  Artificial

<220>
<223>  Designed peptide acting as a vascular endothelial cell binding pe
      ptide

<400>  27

Cys Tyr Leu Thr Met Thr Ala Val Ser Arg Ser Gln Tyr Ser Leu

1               5                   10                  15


<210>  28
<211>  15
<212>  PRT
<213>  Artificial

<220>
<223>  Designed peptide acting as a vascular endothelial cell binding pe
       ptide

<400>  28

Thr Thr Arg Val Trp Leu Asp Gln Met Val Glu Pro Gly Asn Glu
1               5                   10                  15


<210>  29
<211>  15
<212>  PRT
<213>  Artificial

<220>
<223>  Designed peptide acting as a vascular endothelial cell binding pe
       ptide

<400>  29

Asn Leu His Trp Thr Leu Thr Phe Ser Lys Val Pro Thr Gly Glu
1               5                   10                  15


<210>  30
<211>  15
<212>  PRT
<213>  Artificial

<220>
<223>  Designed peptide acting as a vascular endothelial cell binding pe
       ptide

<400>  30

Gly Val Gly Gln Leu Ala Met Thr Leu Ser Gly Arg Gly Ser His
1               5                   10                  15


<210>  31
<211>  15
<212>  PRT
<213>  Artificial

<220>
<223>  Designed peptide acting as a vascular endothelial cell binding pe
       ptide

<400>  31

Ser Arg Thr Pro Asn Asn Ile Met Ala Val Asn Ser His Ser Arg
1               5                   10                  15


<210>  32
<211>  15
<212>  PRT
<213>  Artificial

<220>

<223>  Designed peptide acting as a vascular endothelial cell binding pe
       ptide

<400>  32

Ser Met Val Met Asp Leu Arg Gly Lys Phe Pro Ser Val Arg Val
1               5                   10                  15


<210>  33
<211>  15
<212>  PRT
<213>  Artificial

<220>
<223>  Designed peptide acting as a vascular endothelial cell binding pe
       ptide

<400>  33

Ser Lys Ala Pro Ser Trp Asp Leu Pro Lys Thr Gly Gly Glu Ile
1               5                   10                  15


<210>  34
<211>  15
<212>  PRT
<213>  Artificial

<220>
<223>  Designed peptide acting as a vascular endothelial cell binding pe
       ptide

<400>  34

Ala Arg Arg Pro Ala Val Lys Ala Val Thr Thr Asp Ser Tyr Gly
1               5                   10                  15


<210>  35
<211>  15
<212>  PRT
<213>  Artificial

<220>
<223>  Designed peptide acting as a vascular endothelial cell binding pe
       ptide

<400>  35

Ser Arg Met Pro Met Gly Val His Asp Thr Thr Ala Leu Lys Trp
1               5                   10                  15


<210>  36
<211>  15
<212>  PRT
<213>  Artificial

<220>
<223>  Designed peptide acting as a vascular endothelial cell binding pe
       ptide

<400>  36

Gln Ser Leu Ile Tyr Ser Ser Leu Phe Pro Arg Arg Ser Trp Phe
1               5                   10                  15

<210> 37
<211> 15
<212> PRT
<213> Artificial

<220>
<223> Designed peptide acting as a vascular endothelial cell binding pe
      ptide

<400> 37

Met Thr Leu Ser Met Ala Arg Thr Ala Arg Asp Ile Gly Thr Gly
1               5                   10                  15


<210> 38
<211> 12
<212> PRT
<213> Artificial

<220>
<223> Designed peptide acting as a vascular endothelial cell binding pe
      ptide

<400> 38

Ser Pro Arg Pro Leu Pro Ile Ile Thr Pro Phe Pro
1               5                   10


<210> 39
<211> 12
<212> PRT
<213> Artificial

<220>
<223> Designed peptide acting as a vascular endothelial cell binding pe
      ptide

<400> 39

His Val Thr Ser Tyr Asp Ala Trp Ala Pro Asp Pro
1               5                   10


<210> 40
<211> 12
<212> PRT
<213> Artificial

<220>
<223> Designed peptide acting as a vascular endothelial cell binding pe
      ptide

<400> 40

Asp Tyr Pro Lys Ala Pro Gly Ser His Pro Arg Thr
1               5                   10


<210> 41
<211> 12
<212> PRT
<213> Artificial

<220>
<223> Designed peptide acting as a vascular endothelial cell binding pe
      ptide

<400>  41

Thr Ser Lys Phe Pro Ser Thr Asp Leu Ala Arg Leu
1               5                   10


<210>  42
<211>  12
<212>  PRT
<213>  Artificial

<220>
<223>  Designed peptide acting as a vascular endothelial cell binding pe
       ptide

<400>  42

Val Gly Asn Ser Asp Thr Thr Gly Thr Ser Arg Val
1               5                   10


<210>  43
<211>  12
<212>  PRT
<213>  Artificial

<220>
<223>  Designed peptide acting as a vascular endothelial cell binding pe
       ptide

<400>  43

Ala Thr Trp Ser His His Leu Ser Ser Ala Gly Leu
1               5                   10


<210>  44
<211>  12
<212>  PRT
<213>  Artificial

<220>
<223>  Designed peptide acting as a vascular endothelial cell binding pe
       ptide

<400>  44

Met Gly Tyr Asp Phe Thr Arg Ser Pro Leu Thr Trp
1               5                   10


<210>  45
<211>  12
<212>  PRT
<213>  Artificial

<220>
<223>  Designed peptide acting as a vascular endothelial cell binding pe
       ptide

<400>  45

Met Ala Asn Leu Thr Gly Ser Gly Thr His Asn Leu
1               5                   10


<210>  46
<211>  12
<212>  PRT

<213>  Artificial

<220>
<223>  Designed peptide acting as a vascular endothelial cell binding pe
      ptide

<400>  46

His Leu Ser Ser Arg Asp Leu Ser Leu Thr Ser Leu
1               5                   10


<210>  47
<211>  12
<212>  PRT
<213>  Artificial

<220>
<223>  Designed peptide acting as a vascular endothelial cell binding pe
      ptide

<400>  47

Trp Gly Ile Ser Ile Thr Asn Pro Ala Ala Leu Ser
1               5                   10


<210>  48
<211>  12
<212>  PRT
<213>  Artificial

<220>
<223>  Designed peptide acting as a vascular endothelial cell binding pe
      ptide

<400>  48

Asp Val Asn Lys Leu Leu Leu Arg Leu Pro Val Thr
1               5                   10


<210>  49
<211>  12
<212>  PRT
<213>  Artificial

<220>
<223>  Designed peptide acting as a vascular endothelial cell binding pe
      ptide

<400>  49

Ala Ile Pro Ile Gly Thr Leu Pro Lys Ile His Leu
1               5                   10


<210>  50
<211>  12
<212>  PRT
<213>  Artificial

<220>
<223>  Designed peptide acting as a vascular endothelial cell binding pe
      ptide

<400>  50

Tyr Pro Gln Ala Ser Leu Ser Thr Phe Lys Pro Leu

1                5                   10


<210>  51
<211>  12
<212>  PRT
<213>  Artificial

<220>
<223>  Designed peptide acting as a vascular endothelial cell binding pe
       ptide

<400>  51

Asn Val Phe Arg His Ala Ile Glu Gln Arg Thr Pro
1               5                   10


<210>  52
<211>  12
<212>  PRT
<213>  Artificial

<220>
<223>  Designed peptide acting as a vascular endothelial cell binding pe
       ptide

<400>  52

Ser Phe Tyr Asn Ala Asp Ser Ser Val Ser Arg Leu
1               5                   10


<210>  53
<211>  12
<212>  PRT
<213>  Artificial

<220>
<223>  Designed peptide acting as a vascular endothelial cell binding pe
       ptide

<400>  53

Trp Ser Pro Ser Gln Phe Lys Leu Asp Met Pro His
1               5                   10


<210>  54
<211>  12
<212>  PRT
<213>  Artificial

<220>
<223>  Designed peptide acting as a vascular endothelial cell binding pe
       ptide

<400>  54

Asn Ile Ala Lys Thr Ser Asn Ser Ser His Leu Pro
1               5                   10


<210>  55
<211>  12
<212>  PRT
<213>  Artificial

<220>

<223>  Designed peptide acting as a vascular endothelial cell binding pe
       ptide

<400>  55

Gly Pro Met Leu Asp Met Ala Leu Gly Pro Ala Pro
1               5                   10


<210>  56
<211>  12
<212>  PRT
<213>  Artificial

<220>
<223>  Designed peptide acting as a vascular endothelial cell binding pe
       ptide

<400>  56

Lys Ser Phe Leu Tyr Thr Pro Ala Thr Val Thr His
1               5                   10


<210>  57
<211>  12
<212>  PRT
<213>  Artificial

<220>
<223>  Designed peptide acting as a vascular endothelial cell binding pe
       ptide

<400>  57

Tyr Ala Thr Val His Thr Pro Leu Ala Trp Leu Pro
1               5                   10


<210>  58
<211>  12
<212>  PRT
<213>  Artificial

<220>
<223>  Designed peptide acting as a vascular endothelial cell binding pe
       ptide

<400>  58

Trp Met Pro Thr Lys Pro His Leu His Asn Leu Gly
1               5                   10


<210>  59
<211>  12
<212>  PRT
<213>  Artificial

<220>
<223>  Designed peptide acting as a vascular endothelial cell binding pe
       ptide

<400>  59

Glu Asn Pro Ser Pro Tyr Ile Pro Ile Pro Leu Thr
1               5                   10

<210> 60
<211> 12
<212> PRT
<213> Artificial

<220>
<223> Designed peptide acting as a vascular endothelial cell binding pe
      ptide

<400> 60

Glu Val Pro Leu Thr Gln Phe Leu Trp His Gln Leu
1               5                   10


<210> 61
<211> 12
<212> PRT
<213> Artificial

<220>
<223> Designed peptide acting as a vascular endothelial cell binding pe
      ptide

<400> 61

Met Ser Lys Asp Gln Pro Ser Phe Phe Arg Thr Ser
1               5                   10


<210> 62
<211> 12
<212> PRT
<213> Artificial

<220>
<223> Designed peptide acting as a vascular endothelial cell binding pe
      ptide

<400> 62

His Ala Ala Tyr Leu Val Glu Trp Pro Gly Ala Gly
1               5                   10


<210> 63
<211> 12
<212> PRT
<213> Artificial

<220>
<223> Designed peptide acting as a vascular endothelial cell binding pe
      ptide

<400> 63

Leu Pro Val Gln Leu Pro Val Asn Ile Ser Pro Arg
1               5                   10


<210> 64
<211> 12
<212> PRT
<213> Artificial

<220>
<223> Designed peptide acting as a vascular endothelial cell binding pe
      ptide

<400> 64

Gln Leu Leu Asn Ala Leu Pro Phe Arg Leu Ala Tyr
1               5                   10


<210> 65
<211> 12
<212> PRT
<213> Artificial

<220>
<223> Designed peptide acting as a vascular endothelial cell binding pe
 ptide

<400> 65

Ala Met Gly His Gly Leu Ala Lys Val Thr Thr Arg
1               5                   10


<210> 66
<211> 12
<212> PRT
<213> Artificial

<220>
<223> Designed peptide acting as a vascular endothelial cell binding pe
 ptide

<400> 66

Thr Asn Ser His Ala Val Pro Leu Leu Pro Leu Leu
1               5                   10


<210> 67
<211> 12
<212> PRT
<213> Artificial

<220>
<223> Designed peptide acting as a vascular endothelial cell binding pe
 ptide

<400> 67

Asp Ser Thr Met Met Pro Thr Val Leu Ser Thr Leu
1               5                   10


<210> 68
<211> 12
<212> PRT
<213> Artificial

<220>
<223> Designed peptide acting as a vascular endothelial cell binding pe
 ptide

<400> 68

Val Ser Gly Ser Glu Thr His Thr Met Pro Leu Ala
1               5                   10


<210> 69
<211> 12
<212> PRT

<213>  Artificial

<220>
<223>  Designed peptide acting as a vascular endothelial cell binding pe
      ptide

<400>  69

His Lys Leu Ala Thr Ser Pro Trp Trp Pro Pro Ile
1               5                   10


<210>  70
<211>  12
<212>  PRT
<213>  Artificial

<220>
<223>  Designed peptide acting as a vascular endothelial cell binding pe
      ptide

<400>  70

Ser Thr His His Arg His Tyr His Asp Thr Leu Ala
1               5                   10


<210>  71
<211>  12
<212>  PRT
<213>  Artificial

<220>
<223>  Designed peptide acting as a vascular endothelial cell binding pe
      ptide

<400>  71

Phe Ala Ser Ser His Lys Gln Ile Pro Leu Gln Leu
1               5                   10


<210>  72
<211>  12
<212>  PRT
<213>  Artificial

<220>
<223>  Designed peptide acting as a vascular endothelial cell binding pe
      ptide

<400>  72

Asp Ser Leu Tyr Thr Leu Phe Pro Arg Ser Ala Phe
1               5                   10


<210>  73
<211>  12
<212>  PRT
<213>  Artificial

<220>
<223>  Designed peptide acting as a vascular endothelial cell binding pe
      ptide

<400>  73

His Asn Pro Ile Pro Tyr Thr Ala Pro Leu Leu Phe

1                   5                   10

<210>  74
<211>  12
<212>  PRT
<213>  Artificial

<220>
<223>  Designed peptide acting as a vascular endothelial cell binding pe
       ptide

<400>  74

Met Ile Asn Thr Lys Ile Pro Pro Phe Thr Arg Trp
1               5                   10

<210>  75
<211>  12
<212>  PRT
<213>  Artificial

<220>
<223>  Designed peptide acting as a vascular endothelial cell binding pe
       ptide

<400>  75

His Ala Lys Ser Gln Pro Ile Asn Ser Phe Ser Met
1               5                   10

<210>  76
<211>  10
<212>  PRT
<213>  Artificial

<220>
<223>  Designed peptide acting as a vascular endothelial cell binding pe
       ptide

<400>  76

Val Ile Pro Ile Leu Ser Ile Tyr Thr Ala
1               5                   10

<210>  77
<211>  12
<212>  PRT
<213>  Artificial

<220>
<223>  control sequence

<400>  77

Glu Tyr Asp Leu Ser Thr Ala Gly Gly Ala Ala Ala
1               5                   10

<210>  78
<211>  19
<212>  PRT
<213>  Artificial

<220>
<223>  control sequence

<400> 78

Gly Leu Asp Lys Ser Ser Tyr Arg Ile Asp Thr Phe Ala Ala His Glu
1               5                   10                  15

Val Ala Gly